# DEMANDE DE BREVET EUROPEEN

(11) **EP 2 198 846 A1**
(43) Date de publication de la demande: **23.06.2010**
(21) Numéro de dépôt: 09179892.6
(22) Date de dépôt: 18.12.2009
(51) Int. Cl.: A61K 8/34, A61K 8/37, A61K 8/41, A61K 8/73, A61K 8/92, A61Q 5/10

(54) **Composition de teinture d'oxydation des fibres kératiniques comprenant un corps gras, un épaississant et un précurseur de colorant d'oxydation**

(30) Priorité: 19.12.2008 FR 0807321
(71) Demandeur: L'Oréal, 75008 Paris (FR)
(72) Inventeur: Simonet, Frédéric, 92110, Clichy (FR); Nicolas-Morgantini, Luc, 60810, Rully (FR); Fonseca, Irène, 95370, Montigny Les Cormeilles (FR); Hercouet, Leïla, 93360, Neuilly Plaisance (FR); Audousset, Marie-Pascale, 92600, Asnieres (FR); Ascione, Jean-Marc, 75003, Paris (FR); Laurent, Florence, 92270, Bois Colombes (FR)
(74) Mandataire: Fevrier, Murielle Françoise E.

(57) **Abrégé**

La présente invention a pour objet une composition pour la teinture d'oxydation des fibres kératiniques, et en particulier des fibres kératiniques humaines telles que les cheveux, comprenant,
A) au moins 25 % d'un ou plusieurs corps gras, différents des acides gras
B) un ou plusieurs polymères épaississants,
B) un ou plusieurs précurseurs de colorant,
C) un ou plusieurs agents oxydants et
D) un ou plusieurs agents alcalins, dont au moins un est une amine organique.

La composition conforme à la présente invention se distingue par ses propriétés tinctoriales améliorées.

## Description

La présente demande a pour objet une composition pour la teinture d'oxydation des fibres kératiniques.

Il est connu de teindre les fibres kératiniques et en particulier les cheveux humains avec des compositions tinctoriales contenant des colorants d'oxydation, en particulier des précurseurs de colorants d'oxydation et des modificateurs de coloration.

Les précurseurs de colorants d'oxydation, appelés généralement bases d'oxydation, sont initialement des composés incolores ou faiblement colorés qui, associés à des produits oxydants, peuvent donner naissance par un processus de condensation oxydative à des composés colorés et colorants. Ce sont en général des composés tels que les ortho- ou para-phénylènediamines, les ortho- ou para-aminophénols et les bases hétérocycliques.

On sait également que l'on peut faire varier les nuances obtenues avec ces bases d'oxydation en les associant à des coupleurs ou modificateurs de coloration, ces derniers étant généralement choisis parmi les méta-diaminobenzènes, les méta-aminophénols, les méta-diphénols et certains composés hétérocycliques tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation, appelée également coloration d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agressions extérieures telles que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui comporte en général des zones différemment sensibilisées (c'est-à-dire abîmées) de sa pointe à sa racine.

De nombreuses tentatives ont été faites dans le domaine de la coloration capillaire afin d'améliorer les propriétés tinctoriales à l'aide, par exemple, d'adjuvants. Cependant, le choix de ces adjuvants est délicat dans la mesure où ils doivent améliorer les propriétés tinctoriales des compositions tinctoriales sans nuire aux autres propriétés de ces compositions. En particulier, ces adjuvants ne doivent pas nuire aux propriétés d'éclaircissement des fibres kératiniques et les propriétés d'application de la coloration.

Le but de la présente invention est d'obtenir de nouvelles compositions pour la teinture d'oxydation des fibres kératiniques qui ne présentent pas les inconvénients de l'art antérieur. Plus particulièrement, le but de la présente invention est d'obtenir des compositions de coloration d'oxydation des fibres kératiniques, présentant des propriétés tinctoriales améliorées qui permettent d'atteindre l'éclaircissement souhaitée et qui soient faciles à mélanger et à appliquer, notamment qui ne coulent pas et restent bien localisées au point d'application. Par propriétés tinctoriales améliorées, on entend en particulier une amélioration au niveau de la puissance/intensité et/ou de l'homogénéité de la teinture.

Ainsi, ce but est atteint par la présente invention qui a pour objet une composition pour la teinture d'oxydation des fibres kératiniques, et en particulier des fibres kératiniques humaines telles que les cheveux, comprenant,
A) au moins 25 % d'un ou plusieurs corps gras,
B) un ou plusieurs polymères épaississants,
B) un ou plusieurs précurseurs de colorant,
C) un ou plusieurs agents oxydants et
D) un ou plusieurs agents alcalins.

La composition conforme à la présente invention se distingue par ses propriétés tinctoriales améliorées. En particulier, la composition de l'invention conduit à des colorations qui présentent une bonne puissance et/ou intensité et/ou une bonne homogénéité de la couleur le long de la fibre entre la pointe et la racine des cheveux (appelé aussi la sélectivité de la coloration) et/ou une bonne chromaticité. La composition de l'invention s'applique sans difficulté sur les fibres kératiniques, sans couler. Cette composition permet aussi une plus faible dégradation des fibres kératiniques au cours du processus de coloration.

Enfin, les colorations obtenues à l'aide des compositions de l'invention sont tenaces, et résistent aux diverses agressions extérieures que peuvent subir les fibres kératiniques.

La présente invention a également pour objet un procédé de teinture des fibres kératiniques mettant en oeuvre la composition conforme à l'invention.

La présente invention a aussi pour objet un dispositif à plusieurs compartiments pour la mise en oeuvre de la composition de l'invention.

La présente invention a pour objet l'utilisation pour la teinture d'oxydation des fibres kératiniques de la composition conforme à l'invention.

Ainsi que cela a été mentionné, la composition de l'invention comprend un ou plusieurs corps gras

Par corps gras, on entend, un composé organique insoluble dans l'eau à température ordinaire (25°C) et à pression atmosphérique (760 mm de Hg) (solubilité inférieure à 5% et de préférence à 1% encore plus préférentiellement à 0,1%). Ils présentent dans leur structure un enchaînement d'au moins deux groupements siloxane ou au moins une chaine hydrocarbonée comportant au moins 6 atomes de carbone. En outre, les corps gras sont généralement solubles dans les solvants organiques dans les mêmes conditions de température et de pression, comme par exemple le chloroforme, l'éthanol, le benzène ou le décaméthylcyclopentasiloxane.

Selon la présente invention, la composition comprend au moins 25 % d'un ou plusieurs corps gras différents des acides gras.

Les corps gras sont notamment choisis parmi les alcanes inférieurs, les alcools gras, les esters d'acide gras, les esters d'alcool gras, les huiles en particulier les huiles non siliconées minérales, végétales, animales ou synthétiques, les cires non siliconées et les silicones.

Il est rappelé qu'au sens de l'invention, les alcools et esters gras présentent plus particulièrement un ou plusieurs groupements hydrocarbonés, linéaires ou ramifiés, saturés ou insaturés, comprenant 6 à 30 atomes de carbone, éventuellement substitués, en particulier par un ou plusieurs groupements hydroxyle (en particulier 1 à 4). S'ils sont insaturés, ces composés peuvent comprendre une à trois double-liaisons carbone-carbone, conjuguées ou non.

En ce qui concerne les alcanes inférieurs, ces derniers comprennent de préférence de 6 à 16 atomes de carbone et sont linéaires ou ramifiés, éventuellement cycliques. A titre d'exemple, les alcanes peuvent être choisis parmi l'hexane et le dodécane, les isoparaffines comme l'isohexadécane et l'isodécane.

Comme huiles non siliconées utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 6 à 30 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol® 810, 812 et 818 par la société Dynamit Nobel, l'huile de jojoba, l'huile de beurre de karité ;
- les hydrocarbures de plus de 16 atomes de carbone linéaires ou ramifiés, d'origine minérale ou synthétique, tels que les huiles de paraffine, la vaseline, l'huile de vaseline, les polydécènes, le polyisobutène hydrogéné tel que Parléam® .
- les huiles fluorées partiellement hydrocarbonées ; comme huiles fluorées, on peut citer aussi le perfluorométhylcyclopentane et le perfluoro-1,3 diméthylcyclohexane, vendus sous les dénominations de "FLUTEC® PC1" et "FLUTEC® PC3" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050®" et "PF 5060®" par la Société 3M, ou encore le bromoperfluorooctyle vendu sous la dénomination "FORALKYL®" par la Société Atochem ; le nonafluoro-méthoxybutane et le nonafluoroéthoxyisobutane ; les dérivés de perfluoromorpholine, tels que la 4-trifluorométhyl perfluoromorpholine vendue sous la dénomination "PF 5052®" par la Société 3M.

Les alcools gras utilisables comme corps gras dans la composition de l'invention sont non oxyalkylénés, saturés ou insaturés, linéaires ou ramifiés, et comportent 6 à 30 atomes de carbone et plus particulièrement de 8 à 30 atomes de carbone, on peut citer l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique.

La cire ou les cires non siliconées susceptibles d'être utilisées dans la composition de l'invention sont choisies notamment, parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, les cires de polyéthylène ou de polyoléfines en général.

Les esters sont les esters de mono ou polyacides aliphatiques saturés ou insaturés, linéaires ou ramifiés en C1-C26 et de mono ou polyalcools aliphatiques saturés ou insaturés, linéaires ou ramifiés en C1-C26, le nombre total de carbone des esters étant plus particulièrement supérieur ou égal à 10.

Parmi les monoesters, on peut citer le béhénate de dihydroabiétyle ; le béhénate d'octyldodécyle ; le béhénate d'isocétyle ; le lactate de cétyle ; le lactate d'alkyle en C12-C15 ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, de mirystyle, de stéaryle le stéarate d'hexyle, le stéarate de butyle, le stéarate d'isobutyle ; le malate de dioctyle, le laurate d'hexyle, le laurate de 2-hexyldécyle.

Toujours dans le cadre de cette variante, on peut également utiliser les esters d'acides di ou tricarboxyliques en C4-C22 et d'alcools en C1-C22 et les esters d'acides mono di ou tricarboxyliques et d'alcools di, tri, tétra ou pentahydroxy en C2-C26.

On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate de propylène glycol ; le dicaprate de propylène glycol, l'érucate de tridécyle ; le citrate de triisopropyle ; le citrate de triisotéaryle ; trilactate de glycéryle ; trioctanoate de glycéryle ; le citrate de trioctyldodécyle ; le citrate de trioléyle, le dioctanoate de propylène glycol ; le diheptanoate de néopentyl glycol ; le diisanonate de diéthylène glycol ; et les distéarates de polyéthylène glycol.

Parmi les esters cités ci-dessus, on préfère utiliser les palmitates d'éthyle, d'isopropyle, de myristyle, de cétyle, de stéaryle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle et l'isononanate d'isononyle, l'octanoate de cétyle.

La composition peut également comprendre, à titre d'ester gras, des esters et di-esters de sucres d'acides gras en C6-C30, de préférence en C12-C22. Il est rappelé que l'on entend par « sucre », des composés hydrocarbonés oxygénés qui possèdent plusieurs fonctions alcool, avec ou sans fonction aldéhyde ou cétone, et qui comportent au moins 4 atomes de carbone. Ces sucres peuvent être des monosaccharides, des oligosaccharides ou des polysaccharides.

Comme sucres convenables, on peut citer par exemple le sucrose (ou saccharose), le glucose, le galactose, le ribose, le fucose, le maltose, le fructose, le mannose, l'arabinose, le xylose, le lactose, et leurs dérivés notamment alkylés, tels que les dérivés méthylés comme le méthylglucose.

Les esters de sucres et d'acides gras peuvent être choisis notamment dans le groupe comprenant les esters ou mélanges d'esters de sucres décrits auparavant et d'acides gras en C6-C30, de préférence en C12-C22, linéaires ou ramifiés, saturés ou insaturés. S'ils sont insaturés, ces composés peuvent comprendre une à trois double-liaisons carbone-carbone, conjuguées ou non.

Les esters selon cette variante peuvent être également choisis parmi les mono-, di-, tri- et tétra-esters, les polyesters et leurs mélanges.

Ces esters peuvent être par exemple des oléate, laurate, palmitate, myristate, béhénate, cocoate, stéarate, linoléate, linolénate, caprate, arachidonates, ou leurs mélanges comme notamment les esters mixtes oléopalmitate, oléo-stéarate, palmito-stéarate.

Plus particulièrement, on utilise les mono- et di- esters et notamment les mono- ou di- oléate, stéarate, béhénate, oléopalmitate, linoléate, linolénate, oléostéarate, de saccharose, de glucose ou de méthylglucose.

On peut citer à titre d'exemple le produit vendu sous la dénomination Glucate® DO par la société Amerchol, qui est un dioléate de méthylglucose.

On peut aussi citer à titre d'exemples d'esters ou de mélanges d'esters de sucre d'acide gras :
- les produits vendus sous les dénominations F160, F 140, F110, F90, F70, SL40 par la société Crodesta, désignant respectivement les palmitostéarates de sucrose formés de 73 % de monoester et 27 % de di- et triester, de 61 % de monoester et 39 % de di-, tri-, et tétra-ester, de 52 % de monoester et 48 % de di-, tri-, et tétra-ester, de 45 % de monoester et 55 % de di-, tri-, et tétra-ester, de 39 % de monoester et 61 % de di-, tri-, et tétra-ester, et le mono-laurate de sucrose;
- les produits vendus sous la dénomination Ryoto Sugar Esters par exemple référencés B370 et correspondant au béhénate de saccharose formé de 20 % de monoester et 80 % de di-triester-polyester;
- le mono-di-palmito-stéarate de sucrose commercialisé par la société Goldschmidt sous la dénomination Tegosoft® PSE.

Les silicones utilisables dans la composition de la présente invention, sont des silicones volatiles ou non volatiles, cycliques, linéaires ou ramifiées, modifiées ou non par des groupements organiques, ayant une viscosité de 5.10-6 à 2,5m2/s à 25°C et de préférence 1.10-5 à 1m2/s.

Les silicones utilisables conformément à l'invention peuvent se présenter sous forme d'huiles, de cires, de résines ou de gommes.

De préférence, la silicone est choisie parmi les polydialkylsiloxanes, notamment les polydiméthylsiloxanes (PDMS), et les polysiloxanes organo-modifiés comportant au moins un groupement fonctionnel choisi parmi les groupements poly(oxyalkylène), les groupements aminés et les groupements alcoxy.

Les organopolysiloxanes sont définis plus en détail dans l'ouvrage de Walter NOLL "Chemistry and Technology of Silicones" (1968), Academie Press. Elles peuvent être volatiles ou non volatiles.

Lorsqu'elles sont volatiles, les silicones sont plus particulièrement choisies parmi celles possédant un point d'ébullition compris entre 60°C et 260°C, et plus particulièrement encore parmi:
les polydialkylsiloxanes cycliques comportant de 3 à 7, de préférence de 4 à 5 atomes de silicium. Il s'agit, par exemple, de l'octaméthylcyclotétrasiloxane commercialisé notamment sous le nom de VOLATILE SILICONE® 7207 par UNION CARBIDE ou SILBIONE® 70045 V2 par RHODIA, le décaméthylcyclopentasiloxane commercialisé sous le nom de VOLATILE SILICONE® 7158 par UNION CARBIDE, et SILBIONE® 70045 V5 par RHODIA, ainsi que leurs mélanges.

On peut également citer les cyclocopolymères du type diméthylsiloxanes/ méthylalkylsiloxane, tel que la SILICONE VOLATILE® FZ 3109 commercialisée par la société UNION CARBIDE, de formule : avec

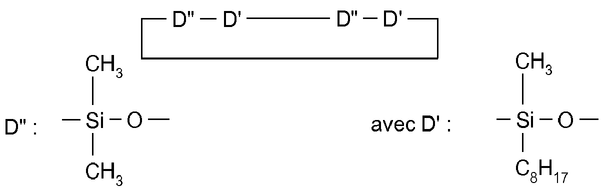

On peut également citer les mélanges de polydialkylsiloxanes cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les polydialkylsiloxanes volatiles linéaires ayant 2 à 9 atomes de silicium et présentant une viscosité inférieure ou égale à 5.10-6m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and Toiletries, Vol. 91, Jan. 76, P. 27-32 - TODD & BYERS "Volatile Silicone fluids for cosmetics".

On utilise de préférence des polydialkylsiloxanes non volatiles, des gommes et des résines de polydialkylsiloxanes, des polyorganosiloxanes modifiés par les groupements organofonctionnels ci-dessus ainsi que leurs mélanges.

Ces silicones sont plus particulièrement choisies parmi les polydialkylsiloxanes parmi lesquels on peut citer principalement les polydiméthylsiloxanes à groupements terminaux triméthylsilyl. La viscosité des silicones est mesurée à 25°C selon la norme ASTM 445 Appendice C.

Parmi ces polydialkylsiloxanes, on peut citer à titre non limitatif les produits commerciaux suivants :
- les huiles SILBIONE® des séries 47 et 70 047 ou les huiles MIRASIL® commercialisées par RHODIA telles que, par exemple l'huile 70 047 V 500 000 ;
- les huiles de la série MIRASIL® commercialisées par la société RHODIA ;
- les huiles de la série 200 de la société DOW CORNING telles que la DC200 ayant viscosité 60 000 mm2/s ;
- les huiles VISCASIL® de GENERAL ELECTRIC et certaines huiles des séries SF (SF 96, SF 18) de GENERAL ELECTRIC.

On peut également citer les polydiméthylsiloxanes à groupements terminaux diméthylsilanol connus sous le nom de dimethiconol (CTFA), tels que les huiles de la série 48 de la société RHODIA.

Dans cette classe de polydialkylsiloxanes, on peut également citer les produits commercialisés sous les dénominations "ABIL WAX® 9800 et 9801" par la société GOLDSCHMIDT qui sont des polydialkyl (C1-C20) siloxanes.

Les gommes de silicone utilisables conformément à l'invention sont notamment des polydialkylsiloxanes, de préférence des polydiméthylsiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécane ou leurs mélanges.

Des produits plus particulièrement utilisables conformément à l'invention sont des mélanges tels que :
- les mélanges formés à partir d'un polydiméthylsiloxane hydroxylé en bout de chaîne, ou diméthiconol (CTFA) et d'un polydiméthylsiloxane cyclique également appelé cyclométhicone (CTFA) tel que le produit Q2 1401 commercialisé par la société DOW CORNING ;
- les mélanges d'une gomme polydiméthylsiloxane et d'une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10-6m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les motifs :
R2SiO2/2, R3SiO1/2, RSiO3/2 et SiO4/2
dans lesquelles R représente un alkyl possédant 1 à 16 atomes de carbone. Parmi ces produits, ceux particulièrement préférés sont ceux dans lesquels R désigne un groupe alkyle inférieur en C1-C4, plus particulièrement méthyle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Outre, les silicones décrites ci-dessus les silicones organomodifiées peuvent être des polydiaryl siloxanes, notamment des polydiphénylsiloxanes, et des polyalkyl-arylsiloxanes fonctionnalisés par les groupes organofonctionnels mentionnés précédemment.

Les polyalkylarylsiloxanes sont particulièrement choisis parmi les polydiméthyl/méthylphénylsiloxanes, les polydiméthyl/diphénylsiloxanes linéaires et/ou ramifiés de viscosité allant de 1.10-5 à 5.10-2m²/s à 25°C.

Parmi ces polyalkylarylsiloxanes, on peut citer à titre d'exemple les produits commercialisés sous les dénominations suivantes :
- les huiles SILBIONE® de la série 70 641 de RHODIA;
- les huiles des séries RHODORSIL® 70 633 et 763 de RHODIA ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Parmi les silicones organomodifiées, on peut citer les polyorganosiloxanes comportant :
- des groupements polyéthylèneoxy et/ou polypropylèneoxy comportant éventuellement des groupements alkyle en C6-C24 tels que les produits dénommés diméthicone copolyol commercialisé par la société DOW CORNING sous la dénomination DC 1248 ou les huiles SILWET® L 722, L 7500, L 77, L 711 de la société UNION CARBIDE et l'alkyl (C12)-méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C1-C4 ;
- des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT.

De préférence, les corps gras ne sont ni oxyalkylénés, ni glycérolés.

Plus particulièrement, les corps gras sont choisis parmi les composés liquides ou pâteux à température ambiante et à pression atmosphérique.

De préférence, le corps gras est un composé liquide à la température de 25°C et à la pression atmosphérique.

Les corps gras sont de préférence choisis parmi les alcanes inférieurs, les alcools gras, les esters d'acide gras, les esters d'alcool gras, les huiles en particulier les huiles non siliconées minérales, végétales ou synthétiques, les silicones

Selon un mode de réalisation, le ou les corps gras est ou sont choisis parmi l'huile de vaseline, les polydécènes, les esters d'acides gras ou d'alcools gras, liquides ou leurs mélanges, en particulier, le ou les corps gras de la composition selon l'invention sont non siliconés.

On choisira de préférence les alcanes ou hydrocarbures et les silicones.

La composition selon l'invention comprend au moins 25 % de corps gras. De préférence la concentration en corps gras va de 25 à 80 % ,encore plus préférentiellement de 25 à 65 %, mieux de 30 à 55 % du poids total de la composition.

La composition contient un ou plusieurs polymères épaississants.

Au sens de la présente invention, on entend par polymère épaississant un polymère qui introduit à 1% en poids dans une solution aqueuse ou hydroalcoolique à 30 % d'éthanol, et à pH = 7 ou dans une huile choisie parmi l'huile de vaseline, le myristate d'isopropyle ou le cyclopentadiméthylsiloxane, permet d'atteindre une viscosité d'au moins 100 cps, de préférence au moins 500 cps, à 25°C et à un taux de cisaillement de 1s-1. Cette viscosité peut être mesurée à l'aide d'un viscosimètre cône/plan (Rhéomètre Haake R600 ou analogue).

Les polymères épaississants peuvent être épaississant de la phase aqueuse et/ou de la phase grasse.

Les polymères épaississants peuvent être des polymères ioniques ou non, associatifs ou non.

En ce qui concerne les polymères épaississants non associatifs, il est tout d'abord rappelé qu'au sens de la présente invention, les polymères épaississants non associatifs sont des polymères épaississants ne contenant pas de chaîne grasse en C10-C30.

Epaississants de la phase aqueuse :
A titre de polymères épaississants de phase aqueuse, on peut citer les polymères épaississants à motifs sucres.
Par motif sucre on entend au sens de la présente invention un motif issu d'un carbohydrate de formule Cn(H2O)n-1 ou (CH2O)n pouvant être éventuellement modifié par sustitution, et/ou par oxydation et/ou par déshydratation.

Les motifs sucre pouvant entrer dans la composition des polymères épaississants de l'invention sont de préférence issus des sucres suivants :.glucose.galactose.arabinose.rhamnose.mannose.xylose.fucose.an hydrogalactose.acide galacturonique.acide glucuronique. acide mannuronique.galactose sulfate anhydrogalactose sulfate

On peut notamment citer à titre de polymères épaississants de l'invention :
- les gommes natives telles que :
   a) les exsudats d'arbres ou d'arbustes dont :
      - la gomme arabique ((polymère ramifié de galactose, d'arabinose,de rhamnose et d'acide glucuronique)
      - la gomme ghatti (polymère issu d'arabinose,de galactose, de mannose, de xylose et d'acide glucuronique)
      - la gomme karaya (polymère issu d'acide galacturonique, de galactose, de rhamnose et d'acide glucuronique)
      - la gomme tragacanthe (ou adragante) (polymère d'acide galacturonique, de galactose, de fucose, de xylose et d'arabinose)
   b)les gommes issues d'algues dont :
      - l'agar (polymère issu de galactose et d'anhydrogalactose)
      - les alginates (polymères d'acide mannuronique et d'acide glucuronique)
      - les carraghénanes et les furcelleranes (polymères de galactose sulfate et d'anhydrogalactose sulfate)
   c)les gommes issus de semences ou tubercules dont :
      .la gomme de guar (polymère de mannose et de galactose)
      -la gomme de caroube (polymère de mannose et de galactose)
      -la gomme de fenugrec (polymère de mannose et de galactose)
      -la gomme de tamarin (polymère de galactose, de xylose et de glucose)
      .la gomme de konjac (polymère de glucose et mannose)
   d)les gommes microbiennes dont :
      - la gomme de xanthane (polymère de glucose, de mannose acétate, de mannose/acide pyruvique et d'acide glucuronique )
      - la gomme de gellane (polymère de glucose partiellement acylé, de rhamnose et d'acide glucuronique)
      - la gomme de scléroglucane (polymère du glucose)
   e)les extraits de plantes dont :
      - la cellulose (polymère du glucose)
      - l'amidon (polymère du glucose)

Ces polymères peuvent être modifiés par voie physique ou chimique. A titre de traitement physique on peut citer notamment la température.

A titre de traitements chimiques on peut citer les réactions d'estérification, d'étherification, d'amidification, d'oxydation. Ces traitements permettent de conduire à des polymères qui peuvent être notamment non ioniques, anioniques ou amphotères.

De préférence ces traitements chimiques ou physiques sont appliqués sur les gommes de guar, les gommes de caroube, les amidons et les celluloses.

Les gommes de guar non-ioniques utilisables selon l'invention peuvent être modifiées par des groupements hydroxylakyle en C1-C6.

Parmi les groupements hydroxyalkyle, on peut mentionner à titre d'exemple, les groupements hydroxyméthyle, hydroxyéthyle, hydroxypropyle et hydroxybutyle.

Ces gommes de guar sont bien connues de l'état de la technique et peuvent par exemple être préparées en faisant réagir des oxydes d'alcènes correspondants tel que par exemple des oxydes de propylène avec la gomme de guar de façon à obtenir une gomme de guar modifiée par des groupements hydroxypropyle.

Le taux d'hydroxyalkylation varie de préférence de 0,4 à 1,2 et correspond au nombre de molécules d'oxyde d'alkylène consommé par le nombre de fonctions hydroxyle libres présentes sur la gomme de guar.

De telles gommes de guar non-ioniques éventuellement modifiées par des groupements hydroxyalkyle sont par exemple vendues sous les dénominations commerciales JAGUAR HP8, JAGUAR HP60 et JAGUAR HP120 par la société RHODIA CHIMIE.

Les molécules d'amidons utilisés dans la présente invention peuvent avoir comme origine botanique les céréales ou encore les tubercules. Ainsi, les amidons sont par exemple choisis parmi les amidons de maïs, de riz, de manioc, d'orge, de pomme de terre, de blé, de sorgho, de pois.

Les amidons peuvent être modifiés par voie chimique ou physique : notamment par une ou plusieurs des réactions suivantes : prégélatinisation, oxydation, réticulation, estérification, éthérification, amidification, traitements thermiques.

De manière plus particulière, ces réactions peuvent être réalisées de la façon suivante :
- prégélatinisation en faisant éclater les granules d'amidon (par exemple séchage et cuisson dans un tambour sécheur) ;
- oxydation par des oxydants forts conduisant à l'introduction de groupes carboxyle dans la molécule d'amidon et à la dépolymèrisation de la molécule d'amidon (par exemple en traitant une solution aqueuse d'amidon par l'hypochlorite de sodium) ;
- réticulation par des agents fonctionnels capables de réagir avec les groupes hydroxyle des molécules d'amidon qui vont ainsi être liées entre elles (par exemple avec des groupes glyceryl et/ou phosphate) ;
- estérification en milieu alcalin pour le greffage de groupes fonctionnels, notamment acyl en en C1-C6 (acétyl), hydroxyalkylés en C1-C6 (hydroxyéthyl, hydroxypropyl), carboxyméthyl, octénylsuccinique.

On peut notamment obtenir par réticulation avec des composés phophorés des phosphates de monoamidon ( du type Am-O-PO-(OX)2), des phosphates de diamidon ( du type Am-O-PO-(OX)-O-Am) ou même de triamidon (du type Am-O-PO- (O-Am)2) ou leurs mélanges (Am signifiant amidon).

X désigne notamment les métaux alcalins (par exemple sodium ou potassium), les métaux alcalinoterreux (par exemple calcium, magnésium), les sels d'ammoniaque, les sels d'amines comme ceux de la monoéthanolamine, la diéthanolamine, la triéthanolamine, l'amino-3 propanediol-1,2, les sels ammoniums issus des aminoacides basiques tels que la lysine, l'arginine, la sarcosine, l'ornithine, la citrulline.

Les composés phosphorés peuvent être par exemple du tripolyphosphate de sodium, de l'orthophosphate de sodium, de l'oxychlorure de phosphore ou du trimétaphosphate de sodium.

On utilisera préférentiellement des phosphates de diamidon ou des composés riches en phosphate de diamidon comme le produit proposé sous les références PREJEL VA-70-T AGGL (phosphate de diamidon de manioc hydroxypropylé gélatinisé) ou PREJEL TK1 (phosphate de diamidon de manioc gélatinisé) ou PREJEL 200 (phosphate de diamidon de manioc acétylé gélatinisé) par la Société AVEBE ou STRUCTURE ZEA de NATIONAL STARCH (phosphate de diamidon de maïs gélatinisé).

Un amidon préféré est un amidon ayant subi au moins une modification voie chimique telle qu'au moins une estérification.

Selon l'invention, on peut aussi utiliser des amidons amphotères, ces amidons amphotères comprennent un ou plusieurs groupements anioniques et un ou plusieurs groupements cationiques. Les groupements anioniques et cationiques peuvent être liés au même site réactif de la molécule d'amidon ou à des sites réactifs différents; de préférence ils sont liés au même site réactif. Les groupements anioniques peuvent être de type carboxylique, phosphate ou sulfate et de préférence carboxylique. Les groupements cationiques peuvent être de type amine primaire, secondaire, tertiaire ou quaternaire.

Les amidons amphotères sont notamment choisis parmi les composés de formules suivantes : 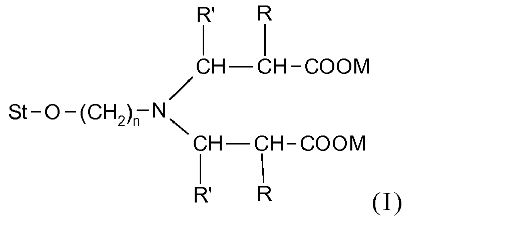 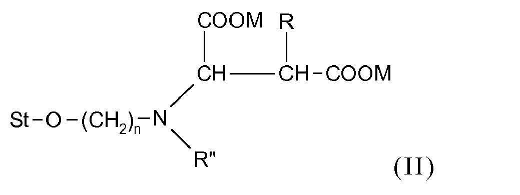 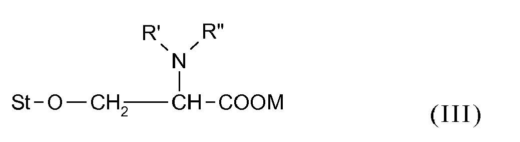 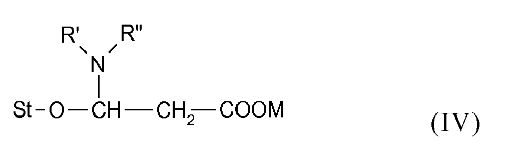 formules dans lesquelles :
St-O représente une molécule d'amidon,
R, identique ou différent, représente un atome d'hydrogène ou un radical méthyle,
R', identique ou différent, représente un atome d'hydrogène, un radical méthyle ou un groupement -COOH,
n est un entier égal à 2 ou 3,
M, identique ou différent, désigne un atome d'hydrogène, un métal alcalin ou alcalinoterreux tels que Na, K, Li, NH4 , un ammonium quaternaire ou une amine organique,
R" représente un atome d'hydrogène ou un radical alkyle ayant de 1 à 18 atomes de carbone.

Ces composés sont notamment décrits dans les brevets US 5,455,340 et US 4,017,460 qui sont inclus à titre de référence.

Les molécules d'amidons peuvent être issues de toutes les sources végétales d'amidon telles que notamment le maïs, la pomme de terre, l'avoine, le riz, le tapioca, le sorgho, l'orge ou le blé. On peut également utiliser les hydrolysats des amidons cités ci-dessus. L'amidon est de préférence issu de la pomme de terre.

On utilise particulièrement les amidons de formules (II) ou (III). On utilise plus particulièrement les amidons modifiés par de l'acide 2-chloroéthyl aminodipropionique, c'est à dire les amidons de formule (II) ou (III) dans lesquelles R, R', R" et M représentent un atome d'hydrogène et n est égal à 2. L'amidon amphotère préféré est un chloroéthylamidodipropionate d'amidon.

Comme indiqué précédemment, les dérivés de celluloses peuvent être notamment anioniques, amphotères ou non-ioniques.

Parmi ces dérivés, on distingue les éthers de celluloses, les esters de celluloses et les esters éthers de celluloses.

Parmi les esters de celluloses, on trouve les esters inorganiques de cellulose (nitrates, sulfates ou phosphates de cellulose...), les esters organiques de cellulose (monoacétates, triacétates, amidopropionates, acétatebutyrates, acétatepropionates ou acétatetrimellitates de cellulose....) et les esters mixtes organique/inorganique de cellulose tels que les acétatebutyratesulfates et les acétatepropionatesulfates de cellulose. Parmi les esters éthers de cellulose, on peut citer les phtalates d'hydroxypropylméthylcellulose et les sulftates d'éthylcellulose.

Parmi les éthers de cellulose non ioniques, on peut citer les alkylcelluloses telles que les méthylcelluloses et les éthylcelluloses (par exemple Ethocel standard 100 Premium de DOW CHEMICAL) ; les hydroxyalkylcelluloses telles que les hydroxyméthylcelluloses, les hydroxyéthylcelluloses(par exemple Natrosol 250 HHR proposé par AQUALON) et les hydroxypropylcelluloses (par exemple Klucel EF d'AQUALON); les celluloses mixtes hydroxyalkyl-alkylcelluloses telles que les hydroxypropyl-méthylcelluloses(par exemple Methocel E4M de DOW CHEMICAL), les hydroxyéthyl-méthylcelluloses, les hydroxyéthyl-éthylcelluloses(par exemple Bermocoll E 481 FQ d'AKZO NOBEL) et les hydroxybutyl-méthylcelluloses.

Parmi les éthers de cellulose anioniques, on peut citer les carboxyalkylcelluloses et leurs sels. A titre d'exemple, on peut citer les carboxyméthylcelluloses, les carboxyméthylméthylcelluloses (par exemple Blanose 7M de la société AQUALON) et les carboxyméthylhydroxyéthylcelluloses et leurs sels de sodium.

Parmi les polymères épaississants non associatifs sans motifs sucre utilisables, on peut citer les homopolymères ou copolymères d'acide acrylique ou méthacryliques réticulés, homopolymères réticulés d'acide 2-acrylamido-2-méthyl-propane sulfonique et leurs copolymères réticulés d'acrylamide, les homopolymères d'acrylate d'ammonium ou les copolymères d'acrylate d'ammonium et d'acrylamide seuls ou en mélanges.

Une première famille de polymères épaississants non associatifs convenable est représentée par les homopolymères d'acide acrylique réticulés.

Parmi les homopolymères de ce type, on peut citer ceux réticulés par un éther allylique d'alcool de la série du sucre, comme par exemple les produits vendus sous les noms CARBOPOLS 980, 981, 954, 2984 et 5984 par la société NOVEON ou les produits vendus sous les noms SYNTHALEN M et SYNTHALEN K par la société 3 VSA.

Les polymères épaississants non associatifs peuvent être aussi des copolymères d'acide (méth)acryliques réticulés tels que le polymère vendu sous la dénomination AQUA SF1 par la société NOVEON.

Les polymères épaississants non associatifs peuvent être choisis parmi les homopolymères réticulés d'acide 2-acrylamido-2-méthylpropane sulfonique et leurs copolymères réticulés d'acrylamide.

En ce qui concerne ces homopolymères et copolymères, qui peuvent être partiellement ou totalement neutralisés, on peut citer les polymères comprenant de 90 à 99,9% en poids, par rapport au poids total du polymère, de motifs de formule (j) suivante : 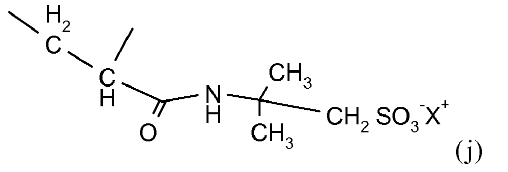 dans laquelle X+ désigne un cation ou un mélange de cations, ou un proton.

Plus particulièrement les cations sont choisis parmi les métaux alcalins (comme le sodium, le potassium), les ions ammonium substitués ou non par un à trois radicaux alkyle, identiques ou différents, comprenant 1 à 6 atomes de carbone, éventuellement porteur d'au moins un radical hydroxyle, les cations dérivant de la N-méthyl-glucamine, d'acides aminés basiques comme l'arginine et la lysine. De préférence, le cation est un ion ammonium ou sodium.

Par ailleurs, le polymère comprend de 0,01 à 10% en poids, par rapport au poids total du polymère, de motifs réticulants provenant d'au moins un monomère ayant au moins deux insaturation éthyléniques (double liaison carbone-carbone).

Les monomères de réticulation ayant au moins deux insaturations éthyléniques sont choisis par exemple parmi l'éther diallylique, le triallylcyanurate, le diallylmaléate, le (méth)acrylate d'allyle, le dipropylèneglycol-diallyléther, les polyglycol-diallyléthers, le triéthylèneglycol-divinyléther, l'hydroquinone-diallyl-éther, le tétrallyloxéthanoyle, le di(méth)acrylate de tétra- ou di-éthylèneglycol, la triallylamine, la tétraallyléthylènediamine le triméthylolpropane-diallyléther, le triméthylolpropane triacrylate, le méthylène-bis(méth)acrylamide ou le divinylbenzène, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

Pour plus de détail au sujet de ces polymères, on pourra se reporter au document EP 815828.

Parmi les copolymères réticulés d'acide 2-acrylamido-2-méthyl-propane sulfonique et d'acrylamide partiellement ou totalement neutralisés, on peut citer en particulier le produit décrit dans l'exemple 1 du document EP 503 853 et l'on pourra se reporter à ce document pour ce qui a trait à ces polymères.

La composition peut de même comprendre, à titre de polymères épaississants non associatifs, les homopolymères d'acrylate d'ammonium ou les copolymères d'acrylate d'ammonium et d'acrylamide.

A titre d'exemples d'homopolymères d'acrylate d'ammonium, on peut citer le produit vendu sous le nom MICROSAP PAS 5193 par la société HOECHST. Parmi les copolymères d'acrylate d'ammonium et d'acrylamide, on peut citer le produit vendu sous le nom BOZEPOL C NOUVEAU ou le produit PAS 5193 vendus par la société HOECHST. On pourra notamment se référer aux documents FR 2 416 723, US 2798053 et US 2923692 pour ce qui a trait à la description et à la préparation de tels composés.

Parmi les agents épaississants, on peut aussi les systèmes épaississants à base de polymères associatifs bien connus de l'homme de l'art et notamment de nature non ionique, anionique, cationique ou amphotère.

Il est rappelé que les polymères associatifs sont des polymères capables, dans un milieu aqueux, de s'associer réversiblement entre eux ou avec d'autres molécules.

Leur structure chimique comprend plus particulièrement au moins une zone hydrophile et au moins une zone hydrophobe.

Par groupement hydrophobe, on entend un radical ou polymère à chaîne hydrocarbonée, saturée ou non, linéaire ou ramifiée, comprenant au moins 10 atomes de carbone, de préférence de 10 à 30 atomes de carbone, en particulier de 12 à 30 atomes de carbone et plus préférentiellement de 18 à 30 atomes de carbone.

Préférentiellement, le groupement hydrocarboné provient d'un composé monofonctionnel. A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.

Parmi les polymères associatifs de type anionique, on peut citer :
- (I) ceux comportant au moins un motif hydrophile, et au moins un motif éther d'allyle à chaîne grasse, plus particulièrement ceux dont le motif hydrophile est constitué par un monomère anionique insaturé éthylénique, plus particulièrement encore par un acide carboxylique vinylique et tout particulièrement par un acide acrylique ou un acide méthacrylique ou les mélanges de ceux ci, et dont le motif éther d'allyle à chaîne grasse correspond au monomère de formule (V) suivante :

   CH2 = C R' CH2 O Bn R (V)
dans laquelle R' désigne H ou CH3, B désigne le radical éthylèneoxy, n est nul ou désigne un entier allant de 1 à 100, R désigne un radical hydrocarboné choisi parmi les radicaux alkyl, arylalkyle, aryle, alkylaryle, cycloalkyle, comprenant de 8 à 30 atomes de carbone, de préférence 10 à 24, et plus particulièrement encore de 12 à 18 atomes de carbone. Un motif de formule (V) plus particulièrement préféré est un motif dans lequel R' désigne H, n est égal à 10, et R désigne un radical stéaryl (C18).

Des polymères associatifs anioniques de ce type sont décrits et préparés, selon un procédé de polymérisation en émulsion, dans le brevet EP-0 216 479.

Parmi ces polymères associatifs anioniques, on préfère particulièrement selon l'invention, les polymères formés à partir de 20 à 60% en poids d'acide acrylique et/ou d'acide méthacrylique, de 5 à 60% en poids de (méth)acrylates d'alkyles inférieurs, de 2 à 50% en poids d'éther d'allyl à chaîne grasse de formule (V), et de 0 à 1% en poids d'un agent réticulant qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

Parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société CIBA sous les dénominations SALCARE SC80® et SALCARE SC90® qui sont des émulsions aqueuses à 30% d'un terpolymère réticulé d'acide méthacrylique, d'acrylate d'éthyle et de steareth-10-allyl éther (40/50/10).
- (II) ceux comportant au moins un motif hydrophile de type acide carboxylique insaturé oléfinique, et au moins un motif hydrophobe de type ester d'alkyl (C10-C30) d'acide carboxylique insaturé.
   De préférence, ces polymères sont choisis parmi ceux dont le motif hydrophile de type acide carboxylique insaturé oléfinique correspond au monomère de formule (VI) suivante : 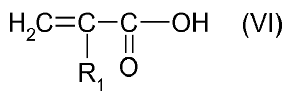
dans laquelle, R1 désigne H ou CH3 ou C2H5, c'est-à-dire des motifs acide acrylique, acide méthacrylique ou acide éthacrylique, et dont le motif hydrophobe de type ester d'alkyl (C10-C30) d'acide carboxylique insaturé correspond au monomère de formule (VII) suivante : 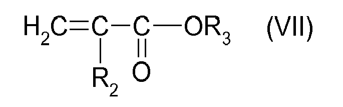 dans laquelle, R2 désigne H ou CH3 ou C2H5 (c'est-à-dire des motifs acrylates, méthacrylates ou éthacrylates) et de préférence H (motifs acrylates) ou CH3 (motifs méthacrylates), R3 désignant un radical alkyle en C10-C30, et de préférence en C12-C22.

Des esters d'alkyles (C10-C30) d'acides carboxyliques insaturés conformes à l'invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.

Des polymères anioniques de ce type sont par exemple décrits et préparés, selon les brevets US-3 915 921 et 4 509 949.

Parmi ce type de polymères associatifs anioniques, on utilisera plus particulièrement des polymères formés à partir d'un mélange de monomères comprenant :
essentiellement de l'acide acrylique,
un ester de formule (VII) décrite ci-dessus et dans laquelle R2 désigne H ou CH3, R3 désignant un radical alkyle ayant de 12 à 22 atomes de carbone, et
   (iii) un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

Parmi ce type de polymères associatifs anioniques, on utilisera plus particulièrement ceux constitués de 95 à 60% en poids d'acide acrylique (motif hydrophile), 4 à 40% en poids d'acrylate d'alkyles en C10-C30 (motif hydrophobe), et 0 à 6% en poids de monomère polymérisable réticulant, ou bien ceux constitués de 98 à 96% en poids d'acide acrylique (motif hydrophile), 1 à 4% en poids d'acrylate d'alkyles en C10-C30 (motif hydrophobe), et 0,1 à 0,6% en poids de monomère polymérisable réticulant tel que ceux décrits précédemment.

Parmi lesdits polymères ci-dessus, on préfère tout particulièrement selon la présente invention, les produits vendus par la société GOODRICH sous les dénominations commerciales PEMULEN TR1®, PEMULEN TR2®, CARBOPOL 1382®, et encore plus préférentiellement le PEMULEN TR1®, et le produit vendu par la société S.E.P.P.I.C. sous la dénomination COATEX SX®.

On peut aussi citer les polymères qui, outre les monomères de formule (VI) et de formule (VII) contiennent un ou plusieurs autres monomères. Ce monomère additionnel peut être notamment un vinyllactame et en particulier la vinylpyrrolidone.

Comme exemple de polymère, on peut citer le terpolymère acide acrylique/méthacrylate de lauryle/vinylpyrrolidone commercialisé sous l'appelation Acrylidone LM par la Société ISP.
- (III) les terpolymères d'anhydride maléique/α-oléfine en C30-C38/ maléate d'alkyle tel que le produit (copolymère anhydride maléique/α-oléfine en C30-C38/maléate d'isopropyle) vendu sous le nom PERFORMA V 1608® par la société NEWPHASE TECHNOLOGIES.
- (IV) les terpolymères acryliques comprenant :
   (a) environ 20% à 70% en poids d'un acide carboxylique à insaturation α,β-monoéthylénique,
   (b) environ 20 à 80% en poids d'un monomère à insaturation α,β-monoéthylénique non-tensio-actif différent de (a),
   (c) environ 0,5 à 60% en poids d'un mono-uréthane non-ionique qui est le produit de réaction d'un tensio-actif monohydrique avec un monoisocyanate à insaturation monoéthylénique,
tels que ceux décrits dans la demande de brevet EP-A-0173109 et plus particulièrement celui décrit dans l'exemple 3, à savoir, un terpolymère acide méthacrylique /acrylate de méthyle/diméthyl métaisopropényl benzyl isocyanate d'alcool béhényle éthoxylé (400E) en dispersion aqueuse à 25%.
- (V) les copolymères comportant parmi leurs monomères un acide carboxylique à insaturation α,β-monoéthylénique et un ester d'acide carboxylique à insaturation α,β-monoéthylénique et d'un alcool gras oxyalkyléné.

Préférentiellement ces composés comprennent également comme monomère un ester d'acide carboxylique à insaturation α,β-monoéthylénique et d'alcool en C1-C4.

A titre d'exemple de ce type de composé on peut citer l'ACULYN 22® vendu par la société ROHM et HAAS, qui est un terpolymère acide méthacrylique/acrylate d'éthyle/méthacrylate de stéaryle oxyalkyléné.

VI)Les polymères amphiphiles comportant au moins un monomère à insaturation éthylènique à groupement sulfonique, sous forme libre ou partiellement ou totalement neutralisée et comprenant au moins une partie hydrophobe. Ces polymères peuvent être réticulés ou non-réticulés. Ils sont de préférence réticulés.

Les monomères à insaturation éthylènique à groupement sulfonique sont choisis notamment parmi l'acide vinylsulfonique, l'acide styrènesulfonique, les acides (méth)acrylamido(C₁-C₂₂)alkylsulfoniques, les acides N-(C₁-C₂₂)alkyl(méth)acrylamido(C₁-C₂₂)alkylsulfoniques comme l'acide undécyl-acrylamido-méthane-sulfonique ainsi que leurs formes partiellement ou totalement neutralisées.

Plus préférentiellement, on utilisera les acides (méth)acrylamido(C₁-C₂₂) alkylsulfoniques tels que par exemple l'acide acrylamido-méthane-sulfonique, l'acide acrylamido-éthane-sulfonique, l'acide acrylamido-propane-sulfonique, l'acide 2-acrylamido-2-méthylpropane-sulfonique, l'acide méthacrylamido-2-méthylpropane-sulfonique, l'acide 2-acrylamido-n-butane-sulfonique, l'acide 2-acrylamido-2,4,4-triméthylpentane-sulfonique, l'acide 2-méthacrylamido-dodécyl-sulfonique, l'acide 2-acrylamido-2,6-diméthyl-3-heptane-sulfonique ainsi que leurs formes partiellement ou totalement neutralisées.

Plus particulièrement, on utilisera l'acide 2-acrylamido-2-méthylpropane-sulfonique (AMPS) ainsi que ses formes partiellement ou totalement neutralisées.

Les polymères de cette famille peuvent notamment être choisis parmi les polymères amphiphiles statistiques d'AMPS modifiés par réaction avec une n-monoalkylamine ou une di-n-alkylamine en C₆-C₂₂, et tels que ceux décrits dans la demande de brevet WO00/31154 (faisant partie intégrante du contenu de la description). Ces polymères peuvent également contenir d'autres monomères hydrophiles éthyléniquement insaturés choisis par exemple parmi les acides (méth)acryliques, leurs dérivés alkyl substitués en β ou leurs esters obtenus avec des monoalcools ou des mono- ou poly-alkylèneglycols, les (méth)acrylamides, la vinylpyrrolidone, l'anhydride maléique, l'acide itaconique ou l'acide maléique ou les mélanges de ces composés.

Les polymères préférés de cette famille sont choisis parmi les copolymères amphiphiles d'AMPS et d'au moins un monomère hydrophobe à insaturation éthylénique.

Ces mêmes copolymères peuvent contenir en outre un ou plusieurs monomères éthyléniquement insaturés ne comportant pas de chaîne grasse tels que les acides (méth)acryliques, leurs dérivés alkyl substitués en β ou leurs esters obtenus avec des monoalcools ou des mono- ou poly-alkylèneglycols, les (méth)acrylamides, la vinylpyrrolidone, l'anhydride maléique, l'acide itaconique ou l'acide maléique ou les mélanges de ces composés.

Ces copolymères sont décrits notamment dans la demande de brevet EP-A-750899, le brevet US 5089578 et dans les publications de Yotaro Morishima suivantes :
- « Self-assembling amphiphilic polyelectrolytes and their nanostructures - Chinese Journal of Polymer Science Vol. 18, N°40, (2000), 323-336. »
- « Miscelle formation of random copolymers of sodium 2-(acrylamido)-2-methylpropanesulfonate and a non-ionic surfactant macromonomer in water as studied by fluorescence and dynamic light scattering - Macromolecules 2000, Vol. 33, N° 10 - 3694-3704 » ;
- « Solution properties of miscelle networks formed by non-ionic moieties covalently bound to an polyelectrolyte : salt effects on rheological behavior - Langmuir, 2000, Vol.16, N°12, 5324-5332 » ;
- « Stimuli responsive amphiphilic copolymers of sodium 2-(acrylamido)-2-methylpropanesulfonate and associative macromonomers - Polym. Preprint, Div. Polym. Chem. 1999, 40(2), 220-221».

Les monomères hydrophobes à insaturation éthylénique de ces copolymères particuliers sont choisis de préférence parmi les acrylates ou les acrylamides de formule (VIII) suivante : 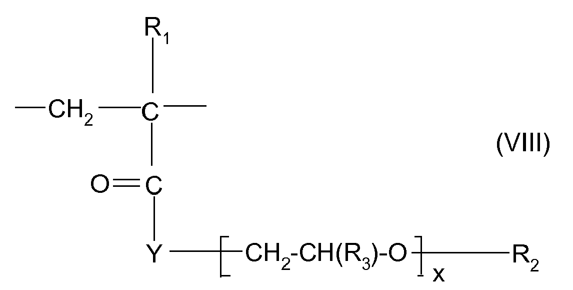 dans laquelle R1 et R3, identiques ou différents, désignent un atome d'hydrogène ou un radical alkyle linéaire ou ramifié en C₁-C₆ (de préférence méthyle) ; Y désigne O ou NH ; R2 désigne un radical hydrocarboné hydrophobe comportant au moins de 8 et plus préférentiellement de 8 à 22 atomes de carbone et encore plus préférentiellement de 8 à 18 atomes de carbone et plus particulièrement de 12 à 18 atomes de carbone ; x désigne un nombre de moles d'oxyde d'alkylène et varie de 0 à 100.

Le radical R2 est choisi de préférence parmi les radicaux alkyles en C₈-C₁₈ linéaires (par exemple n-hexyle, n-octyle, n-décyle, n-hexadécyle, n-dodécyle), ramifiés ou cycliques (par exemple cyclododécane (C₁₂) ou adamantane (C₁₀)) ; les radicaux alkylperfluorés en C₆-C₁₈ (par exemple le groupement de formule -(CH₂)₂-(CF₂)₉-CF₃) ; le radical cholestéryle (C₂₇) ou un reste d'ester de cholestérol comme le groupe oxyhexanoate de cholestéryle ; les groupes polycycliques aromatiques comme le naphtalène ou le pyrène. Parmi ces radicaux, on préfère plus particulièrement les radicaux alkyles linéaires et plus particulièrement le radical n-dodécyle.

Selon une forme particulièrement préférée de l'invention, le monomère de formule (VIII) comporte au moins un motif oxyde d'alkylène (x ≥ 1) et de préférence une chaîne polyoxyalkylénée. La chaîne polyoxyalkylénée, de façon préférentielle, est constituée de motifs oxyde d'éthylène et/ou de motifs oxyde de propylène et encore plus particulièrement constituée de motifs oxyde d'éthylène. Le nombre de motifs oxyalkylénés varie en général de 3 à 100 et plus préférentiellement de 3 à 50 et encore plus préférentiellement de 87 à 25.

Parmi ces polymères, on peut citer :
- les copolymères réticulés ou non réticulés, neutralisés ou non, comportant de 15 à 60% en poids de motifs AMPS et de 40 à 85% en poids de motifs (C₈-C₁₆)alkyl(méth)acrylamide ou de motifs (C₈-C₁₆)alkyl(méth)acrylate par rapport au polymère, tels que ceux décrits dans la demande EP-A750 899 ;
- les terpolymères comportant de 10 à 90% en mole de motifs acrylamide, de 0,1 à 10% en mole de motifs AMPS et de 5 à 80% en mole de
motifs n-(C₆-C₁₈)alkylacrylamide, tels que ceux décrits dans le brevet US-5089578.

On peut également citer les copolymères d'AMPS totalement neutralisé et de méthacrylate de dodécyle ainsi que les copolymères d'AMPS et de n-dodécylméthacrylamide non-réticulés et réticulés, tels que ceux décrits dans les articles de Morishima cités ci-dessus.

On citera plus particulièrement les copolymères constitués de motifs acide 2-acrylamido-2-méthylpropane-sulfonique (AMPS) de formule (IX) suivante : 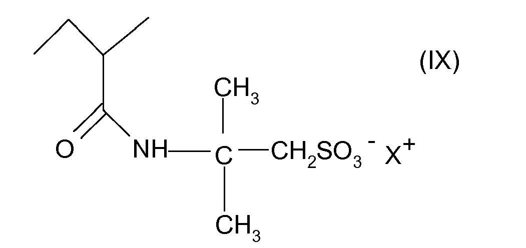 dans laquelle X⁺ est un proton, un cation de métal alcalin, un cation alcalino-terreux ou l'ion ammonium,
et de motifs de formule (X) suivante : 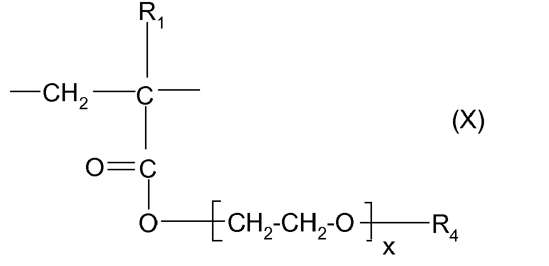 dans laquelle x désigne un nombre entier variant de 3 à 100, de préférence de 3 à 505 à 80 et plus préférentiellement de 87 à 25 ; R1 a la même signification que celle indiquée ci-dessus dans la formule (I) et R4 désigne un alkyle linéaire ou ramifié en C₆-C₂₂ et plus préférentiellement en C₁₂-C₁₈.C₁₀-C₂₂.

Les polymères particulièrement préférés sont ceux pour lesquels x = 25 , R1 désigne méthyle et R4 représente n-dodécyle ; ils sont décrits dans les articles de Morishima mentionnés ci-dessus.

La concentration molaire en % des motifs de formule (IX) et des motifs de formule (X) variera en fonction de l'application cosmétique souhaitée et des propriétés rhéologiques de la formulation recherchées. Elle varie de préférence de 70 à 99% en moles de motifs AMPS et de 1 à 30% en moles de motifs de formule (X) par rapport au copolymère et plus particulièrement de 70 à 90% en moles de motifs AMPS et de 10 à 30% en moles de motifs de formule (X).

Les polymères pour lesquels X+ désigne sodium ou ammonium sont plus particulièrement préférés.

Parmi les polymères associatifs de type cationique, on peut citer :
- (I) les polyuréthanes associatifs cationiques dont la famille a été décrite par la demanderesse dans la demande de brevet français N° 0009609; elle peut être représentée par la formule générale (XI) suivante :

R-X-(P)n-[L-(Y)m]r-L'-(P')p-X'-R' (XI)

dans laquelle :
R et R', identiques ou différents, représentent un groupement hydrophobe ou un atome d'hydrogène ;
X et X', identiques ou différents, représentent un groupement comportant une fonction amine portant ou non un groupement hydrophobe, ou encore le groupement L" ;
L, L' et L", identiques ou différents, représentent un groupement dérivé d'un diisocyanate ;
P et P', identiques ou différents, représentent un groupement comportant une fonction amine portant ou non un groupement hydrophobe ;
Y représente un groupement hydrophile ;
r est un nombre entier compris entre 1 et 100, de préférence entre 1 et 50 et en particulier entre 1 et 25,
n, m, et p valent chacun indépendamment des autres entre 0 et 1000 ; la molécule contenant au moins une fonction amine protonée ou quaternisée et au moins un groupement hydrophobe.

Dans un mode de réalisation préféré de ces polyuréthanes, les seuls groupements hydrophobes sont les groupes R et R' aux extrémités de chaîne.

Une famille préférée de polyuréthanes associatifs cationiques est celle correspondant à la formule (XI) décrite ci-dessus et dans laquelle :
R et R' représentent tous les deux indépendamment un groupement hydrophobe,
X, X' représentent chacun un groupe L",
n et p valent entre 1 et 1000 et
L, L', L", P, P', Y et m ont la signification indiquée ci-dessus.

Une autre famille préférée de polyuréthanes associatifs cationiques est celle correspondant à la formule (XI) ci-dessus dans laquelle :
R et R' représentent tous les deux indépendamment un groupement hydrophobe, X, X' représentent chacun un groupe L", n et p valent 0, et L, L', L", Y et m ont la signification indiquée ci-dessus.

Le fait que n et p valent 0 signifie que ces polymères ne comportent pas de motifs dérivés d'un monomère à fonction amine, incorporé dans le polymère lors de la polycondensation. Les fonctions amine protonées de ces polyuréthanes résultent de l'hydrolyse de fonctions isocyanate, en excès, en bout de chaîne, suivie de l'alkylation des fonctions amine primaire formées par des agents d'alkylation à groupe hydrophobe, c'est-à-dire des composés de type RQ ou R'Q, dans lequel R et R' sont tels que définis plus haut et Q désigne un groupe partant tel qu'un halogénure, un sulfate etc.

Encore une autre famille préférée de polyuréthanes associatifs cationiques est celle correspondant à la formule (XI) ci-dessus dans laquelle :
R et R' représentent tous les deux indépendamment un groupement hydrophobe,
X et X' représentent tous les deux indépendamment un groupement comportant une amine quaternaire,
n et p valent zéro, et
L, L', Y et m ont la signification indiquée ci-dessus.

La masse moléculaire moyenne en nombre des polyuréthanes associatifs cationiques est comprise de préférence entre 400 et 500 000, en particulier entre 1000 et 400 000 et idéalement entre 1000 et 300 000.

Par groupement hydrophobe, on entend un radical ou polymère à chaîne hydrocarbonée, saturée ou non, linéaire ou ramifiée, pouvant contenir un ou plusieurs hétéroatomes tels que P, O, N, S, ou un radical à chaîne perfluorée ou siliconée. Lorsqu'il désigne un radical hydrocarboné, le groupement hydrophobe comporte au moins 10 atomes de carbone, de préférence de 10 à 30 atomes de carbone, en particulier de 12 à 30 atomes de carbone et plus préférentiellement de 18 à 30 atomes de carbone.

Préférentiellement, le groupement hydrocarboné provient d'un composé mono fonctionnel.

A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.

Lorsque X et/ou X' désignent un groupement comportant une amine tertiaire ou quaternaire, X et/ou X' peuvent représenter l'une des formules suivantes : 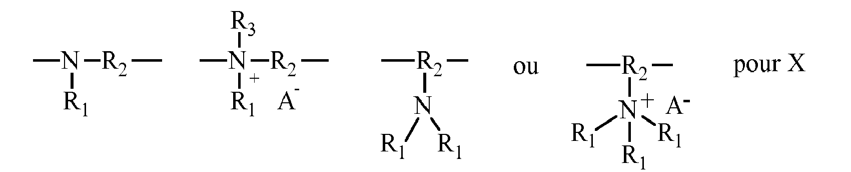 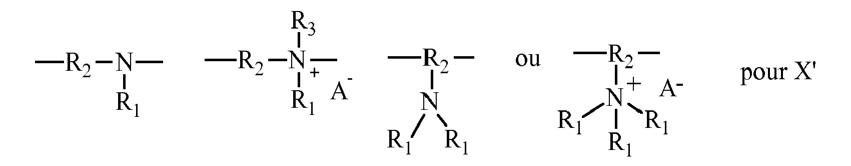 dans lesquelles :
R2 représente un radical alkylène ayant de 1 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O, P ;
R1 et R3, identiques ou différents, désignent un radical alkyle ou alcényle en C1-C30, linéaire ou ramifié, un radical aryle, l'un au moins des atomes de carbone pouvant être remplacé par un hétéroatome choisi parmi N, S, O, P ;
A- est un contre-ion physiologiquement acceptable.

Les groupements L, L' et L" représentent un groupe de formule : 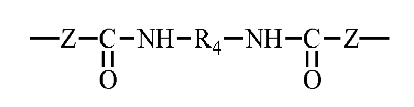 dans laquelle :
Z représente -O-, -S- ou -NH- ; et
R4 représente un radical alkylène ayant de 1 à 20 atomes de carbone, linéaire ou ramifié, comportant ou non un cycle saturé ou insaturé, un radical arylène, un ou plusieurs des atomes de carbone pouvant être remplacé par un hétéroatome choisi parmi N, S, O et P.
   Les groupements P et P', comprenant une fonction amine peuvent représenter au moins l'une des formules suivantes : 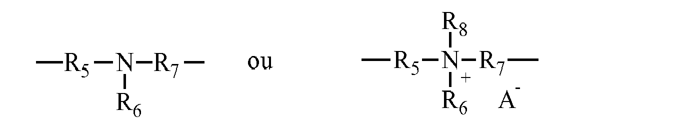 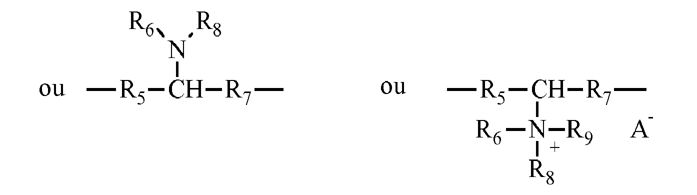 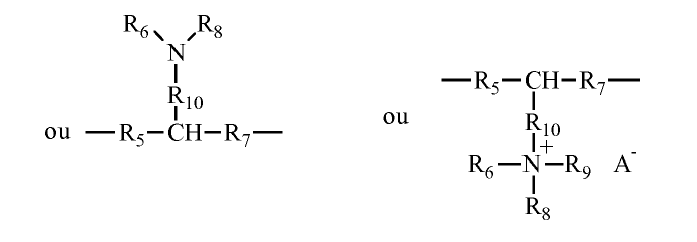
dans lesquelles :
R5 et R7 ont les mêmes significations que R2 défini précédemment;
R6, R8 et R9 ont les mêmes significations que R1 et R3 définis précédemment ;
R10 représente un groupe alkylène, linéaire ou ramifié, éventuellement insaturé et pouvant contenir un ou plusieurs hétéroatomes choisis parmi N, O, S et P,
et A- est un contre-ion physiologiquement acceptable.

En ce qui concerne la signification de Y, on entend par groupement hydrophile, un groupement hydrosoluble polymérique ou non.

A titre d'exemple, on peut citer, lorsqu'il ne s'agit pas de polymères, l'éthylèneglycol, le diéthylèneglycol et le propylèneglycol.

Lorsqu'il s'agit, conformément à un mode de réalisation préféré, d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Les polyuréthanes associatifs cationiques de formule (XI) utilisables selon l'invention sont formés à partir de diisocyanates et de différents composés possédant des fonctions à hydrogène labile. Les fonctions à hydrogène labile peuvent être des fonctions alcool, amine primaire ou secondaire ou thiol 1 donnant, après réaction avec les fonctions diisocyanate, respectivement des polyuréthanes, des polyurées et des polythiourées. Le terme "polyuréthanes" utilisable selon la présente invention englobe ces trois types de polymères à savoir les polyuréthanes proprement dits, les polyurées et les polythiourées ainsi que des copolymères de ceux-ci.

Un premier type de composés entrant dans la préparation du polyuréthane de formule (V) est un composé comportant au moins un motif à fonction amine. Ce composé peut être multifonctionnel, mais préférentiellement le composé est difonctionnel, c'est-à-dire que selon un mode de réalisation préférentiel, ce composé comporte deux atomes d'hydrogène labile portés par exemple par une fonction hydroxyle, amine primaire, amine secondaire ou thiol. On peut également utiliser un mélange de composés multifonctionnels et difonctionnels dans lequel le pourcentage de composés multifonctionnels est faible.

Comme indiqué précédemment, ce composé peut comporter plus d'un motif à fonction amine. Il s'agit alors d'un polymère portant une répétition du motif à fonction amine.

Ce type de composés peut être représenté par l'une des formules suivantes :

HZ-(P)n-ZH,

ou

HZ-(P')p-ZH

dans lesquelles Z, P, P', n et p sont tels que définis plus haut.

A titre d'exemple de composé à fonction amine, on peut citer la N-méthyldiéthanolamine, la N-tert-butyl-diéthanolamine, la N-sulfoéthyldiéthanolamine.

Le deuxième composé entrant dans la préparation du polyuréthane de formule (XI) est un diisocyanate correspondant à la formule :

O=C=N-R4-N=C=O

dans laquelle R4 est défini plus haut.

A titre d'exemple, on peut citer le méthylènediphényl-diisocyanate, le méthylènecyclohexanediisocyanate, l'isophorone-diisocyanate, le toluènediisocyanate, le naphtalènediisocyanate, le butanediisocyanate, l'hexanediisocyanate.

Un troisième composé entrant dans la préparation du polyuréthane de formule (XI) est un composé hydrophobe destiné à former les groupes hydrophobes terminaux du polymère de formule (XI).

Ce composé est constitué d'un groupe hydrophobe et d'une fonction à hydrogène labile, par exemple une fonction hydroxyle, amine primaire ou secondaire, ou thiol.

A titre d'exemple, ce composé peut être un alcool gras, tel que notamment l'alcool stéarylique, l'alcool dodécylique, l'alcool décylique. Lorsque ce composé comporte une chaîne polymérique, il peut s'agir par exemple du polybutadiène hydrogéné alpha-hydroxyle.

Le groupe hydrophobe du polyuréthane de formule (XI) peut également résulter de la réaction de quaternisation de l'amine tertiaire du composé comportant au moins un motif amine tertiaire. Ainsi, le groupement hydrophobe est introduit par l'agent quaternisant. Cet agent quaternisant est un composé de type RQ ou R'Q, dans lequel R et R' sont tels que définis plus haut et Q désigne un groupe partant tel qu'un halogénure, un sulfate etc.

Le polyuréthane associatif cationique peut en outre comprendre une séquence hydrophile. Cette séquence est apportée par un quatrième type de composé entrant dans la préparation du polymère. Ce composé peut être multifonctionnel. Il est de préférence difonctionnel. On peut également avoir un mélange où le pourcentage en composé multifonctionnel est faible.

Les fonctions à hydrogène labile sont des fonctions alcool, amine primaire ou secondaire, ou thiol. Ce composé peut être un polymère terminé aux extrémités des chaînes par l'une de ces fonctions à hydrogène labile.

A titre d'exemple, on peut citer, lorsqu'il ne s'agit pas de polymères, l'éthylèneglycol, le diéthylèneglycol et le propylèneglycol.

Lorsqu'il s'agit d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Le groupe hydrophile noté Y dans la formule (XI) est facultatif. En effet, les motifs à fonction amine quaternaire ou protonée peuvent suffire à apporter la solubilité ou l'hydrodispersibilité nécessaire pour ce type de polymère dans une solution aqueuse.

Bien que la présence d'un groupe Y hydrophile soit facultative, on préfère cependant des polyuréthanes associatifs cationiques comportant un tel groupe.
- (III) le ou les polymères cationiques obtenus par polymérisation d'un mélange de monomères comprenant un ou plusieurs monomères vinyliques substitués par un ou plusieurs groupes amino, un ou plusieurs monomères vinyliques non ioniques hydrophobes, et un ou plusieurs monomères vinyliques associatifs.

En particulier, parmi ces polymères cationiques, on peut notamment citer le composé commercialisé par la société NOVEON sous la dénomination AQUA CC et qui correspond à la dénomination INCI POLYACRYLATE-1 CROSSPOLYMER.

Le POLYACRYLATE-1 CROSSPOLYMER est le produit de la polymérisation d'un mélange de monomères comprenant :
- un méthacrylate de di(alkyl en C1-C4) amino(alkyle en C1-C6),
- un ou plusieurs esters d'alkyle en C1-C30 et de l'acide (méth)acrylique,
- un méthacrylate d'alkyle en C10-C30 polyéthoxylé (20-25 moles de motif oxyde d'éthylène),
- un allyl éther de polyéthylèneglycol/polypropylèneglycol 30/5,
- un méthacrylate d'hydroxy(alkyle en C2-C6), et
- un diméthacrylate d'éthylèneglycol.

(IV) les alkylhydroxyéthylcelluloses quaternisées (cationiques) telles que les produits QUATRISOFT LM 200, QUATRISOFT LM-X 529-18-A, QUATRISOFT LM-X 529-18-B (alkyle en C12) et QUATRISOFT LM-X 529-8 (alkyle en C18) vendus par la société AMERCHOL, les produits CRODACEL QM , CRODACEL QL (alkyle en C12) et CRODACEL QS (alkyle en C18) vendus par la société CRODA et le produit SOFTCAT SL 100 vendu par la société AMERCHOL.

Polymères polyvinyllactames cationiques selon l'invention

Les polymères poly(vinyllactame) cationiques selon l'invention comprennent :
- a) au moins un monomère de type vinyl lactame ou alkylvinyllactame;
- b) au moins un monomère de structures (XII) ou (XIII) suivantes : 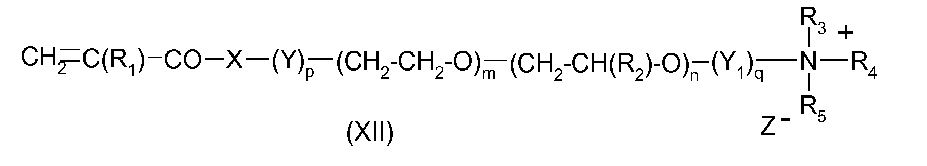 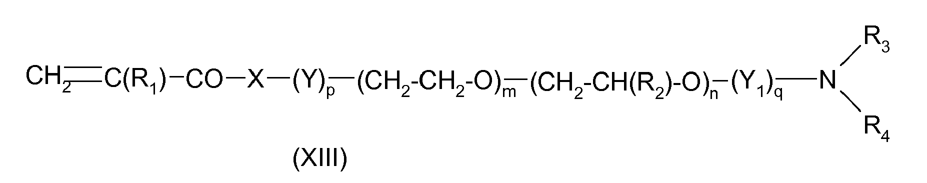
dans lesquelles :
X désigne un atome d'oxygène ou un radical NR6,
R1 et R6 désignent, indépendamment l'un de l'autre, un atome d'hydrogène ou un radical alkyl linéaire ou ramifié en C1-C5,
R2 désigne un radical alkyle linéaire ou ramifié en C1-C4,
R3, R4 et R5 désignent, indépendamment l'un de l'autre, un atome d'hydrogène, un radical alkyle linéaire ou ramifié en C1-C30 ou un radical de formule (XIV) :

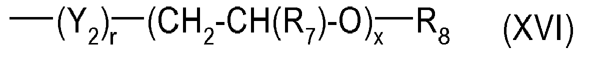

Y , Y1 et Y2 désignent, indépendamment l'un de l'autre, un radical alkylène linéaire ou ramifié en C2-C16,
R7 désigne un atome d'hydrogène, ou un radical alkyle linéaire ou ramifié en C1-C4 ou un radical hydroxyalkyle linéaire ou ramifié en C1-C4,
R8 désigne un atome d'hydrogène ou un radical alkyle linéaire ou ramifié en C1-C30,
p, q et r désignent, indépendamment l'un de l'autre, soit la valeur zéro, soit la valeur 1,
m et n désignent, indépendamment l'un de l'autre, un nombre entier allant de 0 à 100,
x désigne un nombre entier allant de 1 à 100,
Z désigne un anion d'acide organique ou minéral,
sous réserve que :
- l'un au moins des substituants R3, R4, R5 ou R8 désigne un radical alkyle linéaire ou ramifié en C9-C30,
- si m ou n est différent de zéro, alors q est égal à 1,
- si m ou n sont égaux à zéro, alors p ou q est égal à 0.

Les polymères poly(vinyllactame) cationiques selon l'invention peuvent être réticulés ou non réticulés et peuvent aussi être des polymères blocs.

De préférence le contre ion Z- des monomères de formule (XII) est choisi parmi les ions halogénures, les ions phosphates, l'ion méthosulfate, l'ion tosylate.

De préférence R3, R4 et R5 désignent, indépendamment l'un de l'autre, un atome d'hydrogène ou un radical alkyle linéaire ou ramifié en C1-C30.

Plus préférentiellement, le monomère b) est un monomère de formule (XII) pour laquelle, encore plus préférentiellement, m et n sont égaux à zéro.

Le monomère vinyl lactame ou alkylvinyllactame est de préférence un composé de structure (XV) : 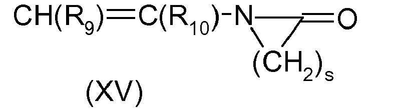 dans laquelle :
s désigne un nombre entier allant de 3 à 6,
R9 désigne un atome d'hydrogène ou un radical alkyle en C1-C5,
R10 désigne un atome d'hydrogène ou un radical alkyle en C1-C5,
sous réserve que l'un au moins des radicaux R9 et R10 désigne un atome d'hydrogène.

Encore plus préférentiellement, le monomère (XV) est la vinylpyrrolidone.

Les polymères poly(vinyllactame) cationiques selon l'invention peuvent également contenir un ou plusieurs monomères supplémentaires, de préférence cationiques ou non ioniques.

A titre de composés plus particulièrement préférés selon l'invention, on peut citer les terpolymères suivants comprenant au moins :
a)-un monomère de formule (XV),
b)-un monomère de formule (XII) dans laquelle p=1, q=0, R3 et R4 désignent, indépendamment l'un de l'autre, un atome d'hydrogène ou un radical alkyle en C1-C5 et R5 désigne un radical alkyle en C9-C24 et
c)-un monomère de formule (XIII) dans laquelle R3 et R4 désignent, indépendamment l'un de l'autre, un atome d'hydrogène ou un radical alkyle en C1-C5.

Encore plus préférentiellement, on utilisera les terpolymères comprenant, en poids, 40 à 95% de monomère (a), 0,1 à 55% de monomère (c) et 0,25 à 50% de monomère (b).

De tels polymères sont décrits dans la demande de brevet WO-00/68282 dont le contenu fait partie intégrante de l'invention.

Comme polymères poly(vinyllactame) cationiques selon l'invention, on utilise notamment les terpolymères vinylpyrrolidone /diméthylaminopropylméthacrylamide / tosylate de dodécyldiméthylméthacrylamidopropylammonium, les terpolymères vinylpyrrolidone /diméthylaminopropylméthacrylamide/ tosylate de cocoyldiméthylméthacrylamidopropylammonium, les terpolymères vinylpyrrolidone / diméthylaminopropylméthacrylamide / tosylate ou chlorure de lauryldiméthylméthacrylamidopropylammonium.

Les polymères associatifs amphotères sont choisis de préférence parmi ceux comportant au moins un motif cationique non cyclique. Plus particulièrement encore, on préfère ceux préparés à partir ou comprenant 1 à 20 moles% de monomère comportant une chaîne grasse, et de préférence 1,5 à 15 moles% et plus particulièrement encore 1,5 à 6 moles%, par rapport au nombre total de moles de monomères.

Les polymères associatifs amphotères préférés selon l'invention comprennent, ou sont préparés en copolymérisant au moins un monomère de formule (XVI) ou (XVII) : 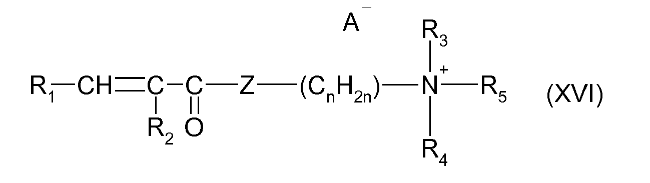 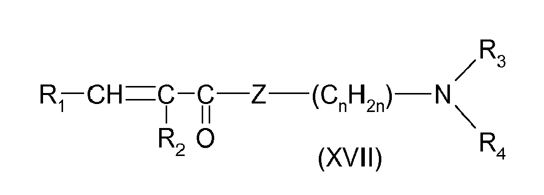 dans lesquelles, R1 et R2, identiques ou différents, représentent un atome d'hydrogène ou un radical méthyle, R3, R4 et R5, identiques ou différents, représente un radical alkyle linéaire ou ramifié ayant de 1 à 30 atomes de carbone,
Z représente un groupe NH ou un atome d'oxygène,
n est un nombre entier de 2 à 5,
A- est un anion issu d'un acide organique ou minéral, tel qu'un anion méthosulfate ou un halogénure tel que chlorure ou bromure;

### 2) au moins un monomère de formule (XVIII)

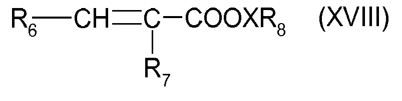

dans laquelle, R6 et R7, identiques ou différents, représentent un atome d'hydrogène ou un radical méthyle;
et

### 3) au moins un monomère de formule (XIX) :

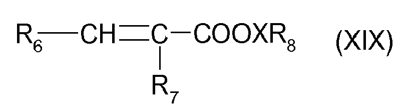

dans laquelle R6 et R7, identiques ou différents, représentent un atome d'hydrogène ou un radical méthyle, X désigne un atome d'oxygène ou d'azote et R8 désigne un radical alkyle linéaire ou ramifié ayant de 1 à 30 atomes de carbone ;
l'un au moins des monomères de formule (XVI), (XVII) ou (XIX) comportant au moins une chaîne grasse.

Les monomères de formule (XVI) et (XVII) sont choisis, de préférence, dans le groupe constitué par :
- le diméthylaminoéthyl méthacrylate, le diméthylaminoéthyl acrylate,
- le diéthylaminoéthyl méthacrylate, le diéthylaminoéthyl acrylate,
- le diméthylaminopropylméthacrylate, le diméthylaminopropyl acrylate,
- le diméthylaminopropyl méthacrylamide, le diméthylaminopropyl acrylamide,
ces monomères étant éventuellement quaternisés, par exemple par un halogénure d'alkyle en C1-C4 ou un sulfate de dialkyle en C1-C4.

Plus particulièrement, le monomère de formule (XVI) est choisi parmi le chlorure d'acrylamidopropyl triméthyl ammonium et le chlorure de méthacrylamidopropyl triméthyl ammonium.

Les monomères de formule (XVIII) de la présente invention sont choisis, de préférence, dans le groupe constitué par l'acide acrylique, l'acide méthacrylique, l'acide crotonique et l'acide méthyl-2 crotonique. Plus particulièrement, le monomère de formule (XVIII) est l'acide acrylique.

Les monomères de formule (XIX) de la présente invention sont choisis, de préférence, dans le groupe constitué par des acrylates ou méthacrylates d'alkyle en C12-C22 et plus particulièrement en C16-C18.

Les monomères constituant les polymères amphotères à chaîne grasse de l'invention sont de préférence déjà neutralisés et/ou quaternisés.

Le rapport du nombre de charges cationiques/charges anioniques est de préférence égal à environ 1.

Les polymères associatifs amphotères de cette famille comprennent de préférence de 1 à 10 % moles du monomère comportant une chaîne grasse (monomère de formule (XVI), (XVII) ou (XIX), et de préférence de 1,5 à 6% moles.

Les polymères associatifs amphotères de cette famille peuvent également contenir d'autres monomères tels que des monomères non ioniques et en particulier tels que les acrylates ou méthacrylates d'alkyle en C1-C4.

Des polymères associatifs amphotères selon l'invention sont par exemple décrits et préparés dans la demande de brevet W09844012.

Parmi les polymères associatifs amphotères selon l'invention, on préfère les terpolymères acide acrylique/chlorure de (méth)acrylamidopropyl triméthyl ammonium/ méthacrylate de stéaryle.

Les polymères associatifs de type non ionique utilisables selon l'invention sont choisis de préférence parmi :
- (a)les copolymères de vinyl pyrrolidone et de monomères hydrophobes à chaîne grasse dont on peut citer à titre d'exemple :
   - les produits ANTARON V216® ou GANEX V216® (copolymère vinylpyrrolidone / hexadécène) vendu par la société I.S.P.
   - les produits ANTARON V220® ou GANEX V220® (copolymère vinylpyrrolidone / eicosène) vendu par la société I.S.P.
- (b) les copolymères de méthacrylates ou d'acrylates d'alkyles en C1-C6 et de monomères amphiphiles comportant au moins une chaîne grasse tels que par exemple le copolymère acrylate de méthyle/acrylate de stéaryle oxyéthyléné vendu par la société GOLDSCHMIDT sous la dénomination ANTIL 208®.
- (c) les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.
- (d) les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.
- (e) les polymères à squelette aminoplaste éther possédant au moins une chaîne grasse, tels que les composés PURE THIX® proposés par la société SUD-CHEMIE.
- (f) les celluloses ou leurs dérivés, modifiés par des groupements comportant au moins une chaîne grasse tels que des groupes alkyls, arylalkyls, alkylaryls ou leurs mélanges où les groupes alkyls sont en C8-et en particulier :
   * les alkylhydroxyéthylcelluloses non-ioniques telles que les produits NATROSOL PLUS GRADE 330 CS et POLYSURF 67 (alkyle en C16) vendus par la société AQUALON
   *les nonoxynylhydroxyéthylcelluloses non-ioniques tels que le produit AMERCELL HM-1500 vendu par la société AMERCHOL ;
   *les alkylcelluloses non-ioniques telles que le produit BERMOCOLL EHM 100 vendu par la société BEROL NOBEL ;
- (g) les dérivés de guar associatifs comme les hydroxypropylguars modifiés par une chaîne grasse tel que le produit ESAFLOR HM 22 (modifié par une chaîne alkyle en C22) vendu par la société LAMBERTI ; le produit MIRACARE XC 95-3 (modifié par une chaîne alkyle en C14) et le produit RE 205-146 (modifié par une chaîne alkyle en C20) vendus par RHODIA CHIMIE ;

De préférence, les polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.

Les polyéthers polyuréthanes non-ioniques à chaîne grasse peuvent être des copolymères triblocs dont la séquence hydrophile est une chaîne polyoxyéthylénée comportant de 50 à 1000 groupements oxyéthylénés. Les polyéthers polyuréthanes non-ioniques comportent une liaison uréthanne entre les séquences hydrophiles, d'où l'origine du nom.

Par extension figurent aussi parmi les polyéthers polyuréthanes non-ioniques à chaîne grasse ceux dont les séquences hydrophiles sont liées aux séquences lipophiles par d'autres liaisons chimiques.

A titre d'exemples de polyéthers polyuréthanes non-ioniques à chaîne grasse utilisables dans l'invention, on peut aussi utiliser aussi le Rhéolate 205® à fonction urée vendu par la société RHEOX ou encore les Rhéolates® 208 , 204 ou 212, ainsi que l'Acrysol RM 184®.

On peut également citer le produit ELFACOS T210® à chaîne alkyle en C12-14 et le produit ELFACOS T212® à chaîne alkyle en C18 de chez AKZO.

Le produit DW 1206B® de chez ROHM & HAAS à chaîne alkyle en C20 et à liaison uréthanne, proposé à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le RHEOLATE® 255, le RHEOLATE® 278 et le RHEOLATE® 244 vendus par la société RHEOX. On peut aussi utiliser le produit DW 1206F et le DW 1206J proposés par la société ROHM & HAAS.

Les polyéthers polyuréthanes utilisables selon l'invention sont en particulier ceux décrits dans l'article de G. Fonnum, J. Bakke et Fk. Hansen - Colloid Polym. Sci 271, 380.389 (1993).

Plus particulièrement encore on préfère utiliser un polyéther polyuréthane susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.

De tels polyéther polyuréthanes sont vendus notamment par la société ROHM & HAAS sous les appellations ACULYN 46® et ACULYN 44® [l'ACULYN 46® est un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81%); l'ACULYN 44® est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%)].

Encore plus préférentiellement le ou les polymères épaississants pour phase aqueuse de l'invention sont choisis parmi les polymères à motifs sucre associatifs ou non et les polymères anioniques acryliques ou méthacryliques associatifs ou non, les polyuréthanes associatifs ou non..

Epaississants de phase grasse.

De préférence, les polymères structurant la phase huileuse via des interactions physiques sont choisis parmi les polyamides, les polyamides siliconés, les mono- ou poly-alkylesters de saccharide ou polysaccharide, les dérivés amides d'aminoacides N-acylés, copolymères comprenant une séquence alkylène ou styrène, ces copolymères pouvant être des polymères di-blocs, tri-bloc, multi-bloc, radial-bloc encore appelés copolymères en étoile, ou encore des polymères en peigne.

### 1) Les polymères portant dans le squelette au moins une séquence cristallisable

Il s'agit encore de polymères solubles ou dispersibles dans l'huile ou phase huileuse par chauffage au-dessus de leur point de fusion pF. Ces polymères sont notamment des copolymères séquencés constitués d'au moins 2 séquences de nature chimique différente dont l'une est cristallisable.

A titre de polymères portant dans le squelette au moins une séquence cristallisable convenant à la mise en oeuvre de l'invention, on peut mentionner :
i). Les polymères définis dans le document US-A-5,156,911 ;
ii). Les copolymères séquencés d'oléfine ou de cyclooléfine à chaîne cristallisable comme ceux issus de la polymérisation séquencée de :
   - cyclobutène, cyclohexène, cyclooctène, norbornène (c'est-à-dire bicyclo(2,2,1)heptène 2), 5-méthylnorbornène, 5-éthylnorbornène, 5,6-diméthyl- norbornène, 5,5,6-triméthyl norbornène, 5-éthylidène-norbornène, 5-phénylnorbornène, 5-benzylnorbornène, 5-vinylnorbornène, 1,4,5,8-diméthano-1,2,3,4,4a,5,8a-octahydronaphtalène, dicyclopentadiène, et leurs mélanges ;
   - avec l'éthylène, le propylène, le 1-butène, le 3-méthyl-1-butène, le 1-hexène, le 4-méthyl-1-pentène, le 1-octène, le 1-décène, le 1-éicosène ou leurs mélanges. Ces copolymères séquencés peuvent être en particulier les copolymères (éthylène/norbornène) blocs et les terpolymères blocs (éthylène/propylène/ éthylidène-norbornène).

On peut aussi utiliser ceux résultants de la copolymérisation séquencée d'au moins 2 α-oléfines en C2-C16 et mieux en C2-C12, tels que ceux cités précédemment et en particulier les bipolymères séquencés d'éthylène et d'1-octène.

Les copolymères présentant au moins une séquence cristallisable, le reste du copolymère étant amorphe (à température ambiante). Ces copolymères peuvent, en outre, présenter deux séquences cristallisables de nature chimique différente. Les copolymères préférés sont ceux qui possèdent à la fois à température ambiante, une séquence cristallisable et une séquence amorphe à la fois hydrophobe et lipophile réparties séquentiellement ; on peut citer par exemple les polymères possédant une des séquences cristallisables et une des séquences amorphes suivantes :
- Séquence cristallisable par nature : a) polyester comme les poly(alkylène téréphtalate), b) polyoléfine comme les polyéthylènes ou polypropylènes.
- Séquence amorphe et lipophile comme les polyoléfines ou copoly(oléfine)s amorphes telles que le poly(isobutylène), le polybutadiène hydrogéné, le poly(isoprène) hydrogéné.

Comme exemple de tels copolymères à séquence cristallisable et à séquence amorphe, on peut citer :
a) les copolymères séquencés poly(δ-caprolactone)-b-poly(butadiène), utilisés de préférence hydrogénés, tels que ceux décrits dans l'article "Melting behavior of poly(δ-caprolactone)-block-polybutadiène copolymers" de S. Nojima, Macromolécules, 32, 3727-3734 (1999).
b) les copolymères séquencés poly(butylènetéréphtalate)-b-poly(isoprène) hydrogénés séquencés ou multiséquencés, cités dans l'article "Study of morphological and mechanical properties of PP/PBT" de B. Boutevin et al.,Polymer Bulletin, 34, 117-123 (1995).
c) les copolymères séquencés poly(éthylène)-b-copoly(éthylène/propylène) cités dans les articles "Morphology of semi-crystalline block copolymers of ethylene- (ethylene-alt-propylene)" de P. Rangarajan et al., Macromolecules, 26, 4640-4645 (1993), et "Polymer agregates with crystalline cores : the system poly(ethylene)-poly(ethylene-propylene)" P. Richter et al., Macromolécules, 30, 1053-1068 25 (1997).
d) les copolymères séquencés poly(éthylène)-b-poly(éthyléthylène) cités dans l'article général "Cristallization in block copolymers" de I.W. Hamley, Advances in Polymer Science, vol 148, 113-137 (1999).

Les polymères semi-cristallins utilisables dans le cadre de l'invention peuvent être non réticulés ou réticulés en partie, du moment que le taux de réticulation ne gêne pas leur dissolution ou dispersion dans la phase huileuse liquide par chauffage au dessus de leur température de fusion. Il peut s'agir alors d'une réticulation chimique, par réaction avec un monomère multifonctionnel lors de la polymérisation. Il peut aussi s'agir d'une réticulation physique qui peut alors être due soit à l'établissement de liaisons type hydrogène ou dipolaire entre des groupes portés par le polymère, comme par exemple les interactions dipolaires entre ionomères carboxylates, ces interactions étant en faible quantité et portées par le squelette du polymère ; soit à une séparation de phase entre les séquences cristallisables et les séquences amorphes portées par le polymère.

De préférence, les polymères semi-cristallins convenant à l'invention sont non réticulés.

A titre d'exemple particulier de polymère semi-cristallin utilisable dans la composition selon l'invention, on peut citer les produits Intelimer® de la société Landec décrits dans la brochure « Intelimer® polymers ». Ces polymères sont sous forme solide à température ambiante (25 °C). Ils sont porteurs de chaînes latérales cristallisables et présentent le monomère tel que défini dans la formule X précédente. On peut citer notamment le « Landec IP22® », ayant une température de fusion pF de 56 °C, qui est un produit visqueux à température ambiante, imperméable, non-collant.

On peut aussi utiliser les polymères semi-cristallins décrits dans les exemples 3, 4, 5, 7, 9 du document US-A-5,156,911, résultant de la copolymérisation d'acide acrylique et d'alkyl(méth)acrylate en C5 à C16 comme ceux résultant de la copolymérisation :
- d'acide acrylique, d'hexadécylacrylate et d'isodécylacrylate dans un rapport 1/16/3,
- d'acide acrylique et de pentadécylacrylate dans un rapport 1/19,
- d'acide acrylique, d'hexadécylacrylate, d'éthylacrylate dans un rapport 2,5/76,5/20,
- d'acide acrylique, d'hexadécylacrylate et de méthylacrylate dans un rapport 5/85/10,
- d'acide acrylique, d'octadécylméthacrylate dans un rapport 2,5/97,5.

On peut aussi utiliser le polymère « Structure O » commercialisé par la société National Starch, tel que celui décrit dans le document US-A-5,736,125, de pF 44 °C, ainsi que les polymères semi-cristallins à chaînes pendantes cristallisables comportant des groupements fluorés tels que décrits dans les exemples 1, 4, 6, 7 et 8 du document WO-A-01/19333.

On peut encore utiliser les polymères semi-cristallins obtenus par copolymérisation d'acrylate de stéaryle et d'acide acrylique ou de NVP ou par copolymérisation de l'acrylate de béhényle et de l'acide acrylique ou de NVP, tels que décrits dans le document US-A-5,519,063 ou EP-A- 0 550 745.

Selon une variante particulière de réalisation, les polymères semi-cristallins convenant à la mise en oeuvre de la présente invention sont notamment les acrylates alkylés, parmi lesquels on peut mentionner les copolymères de LANDEC :
- Doresco IPA 13-1® : poly acrylate de stéaryle, pf de 49 °C et PM de 145000;
- Doresco IPA 13-3® : poly acrylate/ acide méthacrylique, pf de 65 °C et PM de 114000;
- Doresco IPA 13-4® : poly acrylate/ vinyl pirrolidone, pf de 44 °C et PM de 387000;
- Doresco IP A13-5® : poly acrylate / methacrylate d'hydroxyethyle, pf de 47 °C et PM de 397600;
- Doresco IPA 13-6® : poly acrylate de béhényle, pf de 66 °C.

### 2) Les polyamides non siliconés

Les polyamides particuliers utilisés dans la composition selon la présente invention sont de préférence ceux décrits dans le document US-A-5,783,657 de la Société UNION CAMP. La partie de US-A-5,783,657 consacrée à ces polymères est incorporée par référence.

Chacun de ces polyamides satisfait notamment à la formule suivante : 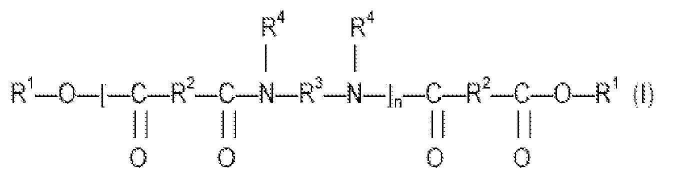 dans laquelle n désigne un nombre entier de motifs amide tel que le nombre de groupes ester représente de 10 % à 50 % du nombre total des groupes ester et amide ; R1 est à chaque occurrence indépendamment un groupe alkyle ou alcényle ayant au moins 4 atomes de carbone et notamment de 4 à 24 atomes de carbone ; R2 représente à chaque occurrence indépendamment un groupe hydrocarboné en C4 à C55 à condition que 50 % au moins des groupes R2 représentent un groupe hydrocarboné en C30 à C55 ; R3 représente à chaque occurrence indépendamment un groupe organique pourvu d'au moins 2 atomes de carbone, d'atomes d'hydrogène et optionnellement d'un ou plusieurs atomes d'oxygène ou d'azote ; et R4 représente à chaque occurrence indépendamment un atome d'hydrogène, un groupe alkyle en C1 à C10 ou une liaison directe à R3 ou à un autre R4 de sorte que l'atome d'azote auquel sont liés à la fois R3 et R4 fasse partie d'une structure hétérocyclique définie par R4-N-R3, avec au moins 50 % des R4 représentant un atome d'hydrogène. En particulier, les groupes ester de ce polyamide représentent de 15 à 40 % du nombre total des groupes ester et amide et au mieux de 20 à 35 %. De plus, n représente avantageusement un nombre entier allant de 1 à 10, et mieux de 1 à 5, bornes incluses.

De préférence, R1 est un groupe alkyle en C12 à C22 et de préférence en C16 à C22. Avantageusement, R2 peut être un groupe hydrocarboné (alkylène) en C10 à C42. De préférence, 50 % au moins et mieux 75 % au moins des R2 sont des groupes ayant de 30 à 42 atomes de carbone. Les autres R2 sont des groupes hydrogénés en C4 à C 19 et de préférence en C4 à C12. De préférence, R3 représente un groupe hydrocarboné en C2 à C36 ou un groupe polyoxyalkyléné et R4 représente un atome d'hydrogène. De préférence, R3 représente un groupe hydrocarboné en C2 à C12. Les groupes hydrocarbonés peuvent être des groupes linéaires, cycliques ou ramifiés, saturés ou insaturés. Par ailleurs, les groupes alkyle et alkylène peuvent être des groupes linéaires ou ramifiés, saturés ou non.

L'épaississement de la phase grasse liquide peut être obtenue à l'aide d'un ou plusieurs polyamides définis ci-dessus. En général, ces polyamides se présentent sous forme de mélanges, ces mélanges pouvant en outre contenir un produit de synthèse correspondant à un polyamide tel que défini ci-dessus avec n valant 0, c'est-à-dire un diester.

Comme polyamide structurant utilisable dans l'invention, on peut encore citer les résines polyamides résultant de la condensation d'un acide di-carboxylique aliphatique et d'un diamine (incluant les composés ayant plus de deux groupes carbonyle et deux groupes amine), les groupes carbonyle et amine de motifs unitaires adjacents étant condensés par une liaison amide. Ces résines polyamides sont notamment celles commercialisées sous la marque Versamid® par les sociétés General Mills, Inc. et Henkel Corp., sous la marque Onamid® notamment Onamid S ou C. Ces résines ont une masse moléculaire moyenne en poids allant de 6000 et 9000. Pour plus d'information sur ces polyamides, on peut se référer aux documents US-A-3,645,705 et US-A-3,148,125. Plus spécialement, on utilise les Versamid® 30 ou 744.

On peut aussi utiliser les polyamides vendus ou fabriqués par la société Arizona sous les références Uni-Rez (2658, 2931, 2970, 2621,2613, 2624, 2665, 1554, 2623, 2662) et le produit vendu sous la référence Macromelt 6212 par la société Henkel. Pour plus d'information sur ces polyamides, on peut se référer au document USA-5500209.

A titre d'exemple de polyamides structurant utilisables dans la composition selon l'invention, on peut encore citer les produits commerciaux vendus ou fabriqués par la société Arizona Chemical sous les noms Uniclear 80 et Uniclear 100. Ils sont vendus respectivement sous forme de gel à 80 % (en matière active) et à 100% (en matière active) dans une huile minérale. Ils ont un point de ramollissement de 88 à 105 °C. Ces produits commerciaux sont un mélange de copolymère d'un diacide en C36 condensé sur l'éthylène diamine, de masse moléculaire moyenne d'environ 6000. Les groupes ester terminaux résultent de l'estérification des terminaisons d'acide restantes par l'alcool cétylique, stéarylique ou leurs mélanges (appelés aussi alcool cétylstéarylique).

### 3) Les polyamides siliconés

Les polymères (homopolymères ou copolymères) de type polyamide siliconé, convenant à la mise en oeuvre de l'invention, possèdent une masse moléculaire moyenne comprise dans l'intervalle allant de 500 à 500 000, et possèdent au moins un groupe comprenant :
- au moins un groupe polyorganosiloxane, comportant de 1 à 1000 unités organosiloxane, dans la chaîne du groupe ou sous forme de greffon, et
- au moins deux groupes capables d'établir des interactions hydrogènes choisis parmi les groupes ester, amide, sulfonamide, carbamate, thiocarabamate, urée, thiourée, oxamido, guanidino, biguanidino, et leurs combinaisons, à condition qu'au moins un de ces groupes soit différent d'un groupe ester,
- le polymère étant solide à température ambiante et soluble dans la phase huileuse à une température variant de 25 à 120 °C.

Les polymères de type polyamide siliconé convenant à la mise en oeuvre de l'invention, et utilisés comme agent structurant de l'huile, peuvent appartenir aux deux familles suivantes :
- des polyorganosiloxanes comprenant au moins deux groupes capables d'établir des interactions hydrogènes, ces deux groupes étant disposés dans la chaîne du polymère, et/ou,
- des polyorganosiloxanes comprenant au moins deux groupes capables d'établir des interactions hydrogènes, ces deux groupes étant disposés sur des greffons ou ramifications.
Les polymères de type polyamide siliconé comportant deux groupes capables d'établir des interactions hydrogène dans la chaîne du polymère peuvent être des polymères comprenant au moins un motif répondant à la première formule suivante : 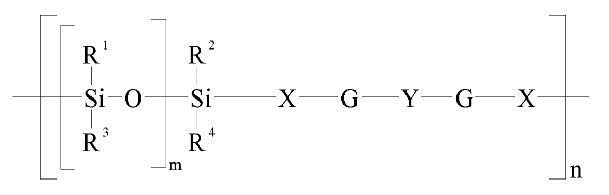 dans laquelle :
1. R1, R2, R3 et R4, identiques ou différents, représentent un groupe choisi parmi :
   - les groupes hydrocarbonés, linéaires, ramifiés ou cycliques, en C1 à C40, saturés ou insaturés, pouvant contenir dans leur chaîne un ou plusieurs atomes d'oxygène, de soufre et/ou d'azote, et pouvant être substitués en partie ou totalement par des atomes de fluor,
   - les groupes aryles en C6 à C10, éventuellement substitués par un ou plusieurs groupes alkyle en C1 à C4,
   - les chaînes polyorganosiloxanes contenant ou non un ou plusieurs atomes d'oxygène, de soufre et/ou d'azote ;
2. les X, identiques ou différents, représentent un groupe alkylène di-yle, linéaire ou ramifié en C1 à C30, pouvant contenir dans sa chaîne un ou plusieurs atomes d'oxygène et/ou d'azote ;
3. Y est un groupe divalent alkylène linéaire ou ramifié, arylène, cycloalkylène, alkylarylène ou arylalkylène, saturé ou insaturé, en C1 à C50, pouvant comporter un ou plusieurs atomes d'oxygène, de soufre et/ou d'azote, et/ou porter comme substituant l'un des atomes ou groupes d'atomes suivants : fluor, hydroxy, cycloalkyle en C3 à C8, alkyle en C1 à C40, aryle en C5 à C10, phényle éventuellement substitué par 1 à 3 groupes alkyle en C1 à C3, hydroxyalkyle en C1 à C3 et amino alkyle en C1 à C6, ou
4. Y représente un groupe répondant à la formule : 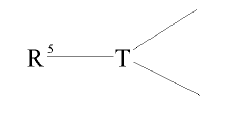 dans laquelle :
   - T représente un groupe hydrocarboné trivalent ou tétravalent, linéaire ou ramifié, saturé ou insaturé, en C3 à C24 éventuellement substitué par une chaîne polyorganosiloxane, et pouvant contenir un ou plusieurs atomes choisis parmi O, N et S, ou T représente un atome trivalent choisi parmi N, P et Al, et
   - R5 représente un groupe alkyle en C1 à C50, linéaire ou ramifié, ou une chaîne polyorganosiloxane, pouvant comporter un ou plusieurs groupes ester, amide, uréthane, thiocarbamate, urée, thiourée et/ou sulfonamide qui peut être lié ou non à une autre chaîne du polymère,
5. les G, identiques ou différents, représentent les groupes divalents choisis parmi : 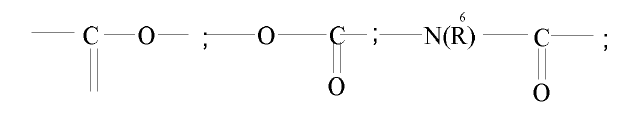 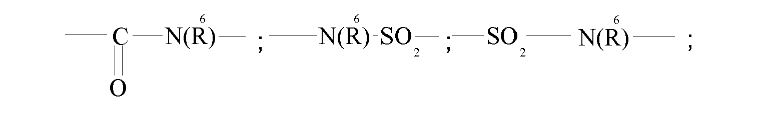 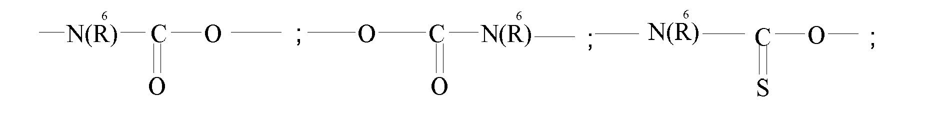 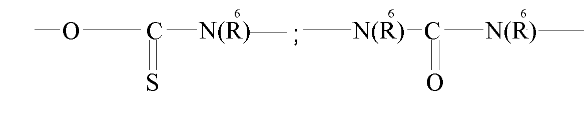 et 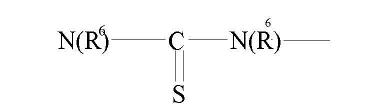 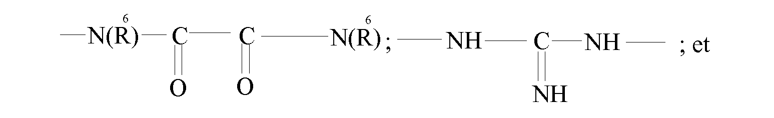 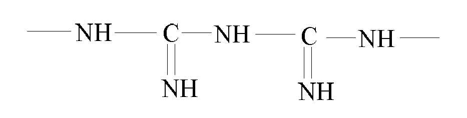 où R6 représente un atome d'hydrogène ou un groupe alkyle, linéaire ou ramifié, en C1 à C20,
6. n est un nombre entier allant de 2 à 500, en particulier de 2 à 200, et m est un nombre entier allant de 1 à 1000, en particulier de 1 à 700 et mieux encore de 6 à 200.

Selon une variante de réalisation, 80 % des R1, R2, R3 et R4, du polymère peuvent être choisis notamment parmi les groupes méthyle, éthyle, phényle et 3,3,3-trifluoropropyle.

Selon une autre variante de réalisation, Y peut représenter divers groupes divalents, comportant éventuellement de plus une ou deux valences libres pour établir des liaisons avec d'autres motifs du polymère ou copolymère. Notamment, Y peut représenter un groupe choisi parmi :
a) les groupes alkylène linéaires en C1 à C20, notamment en C1 à C10;
b) les groupes alkylène ramifiés pouvant comporter des cycles et des insaturations non conjuguées, en C30 à C56 ;
c) les groupes cycloalkylène en C5-C6 ;
d) les groupes phénylène éventuellement substitués par un ou plusieurs groupes alkyle en C1 à C40 ;
e) les groupes alkylène en C1 à C20, comportant de 1 à 5 groupes amides ;
f) les groupes alkylène en C1 à C20, comportant un ou plusieurs substituants, choisis parmi les groupes hydroxyle, cycloalcane en C3 à C8, hydroxyalkyle en C1 à C3 et alkylamines en C1 à C6 ;
g) les chaînes polyorganosiloxane de formule : 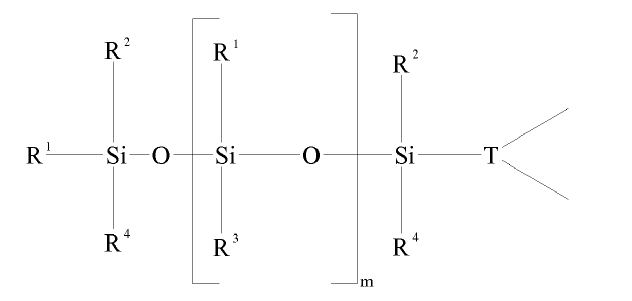 dans laquelle R1, R2, R3 et R4, T et m sont tels que définis ci-dessus; et
h) les chaînes polyorganosiloxanes de formule :

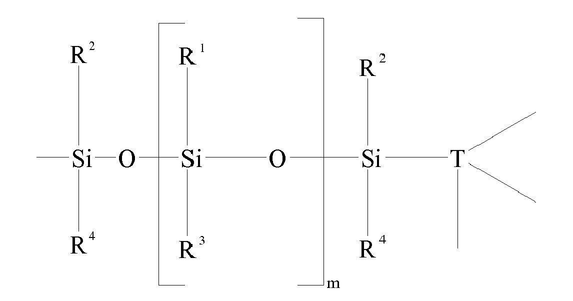

Les polyorganosiloxanes de la seconde famille peuvent être des polymères comprenant au moins un motif répondant à la seconde formule suivante: 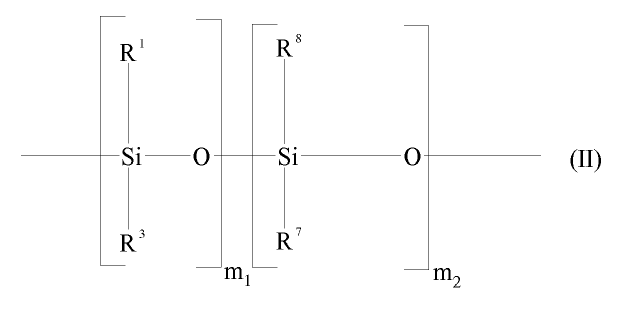 dans laquelle :
- R1 et R3, identiques ou différents, sont tels que définis ci-dessus pour la formule précédente ;
- R7 représente un groupe tel que défini ci-dessus pour R1 et R3, ou représente le groupe de formule -X-G-R9 dans laquelle X et G sont tels que définis ci-dessus pour la formule précédente et R9 représente un atome d'hydrogène ou un groupe hydrocarboné, linéaire, ramifié ou cyclique, saturé ou insaturé, en C1 à C50 comportant éventuellement dans sa chaîne un ou plusieurs atomes choisis parmi O, S et N, éventuellement substitué par un ou plusieurs atomes de fluor et/ou un ou plusieurs groupes hydroxyle, ou un groupe phényle éventuellement substitué par un ou plusieurs groupes alkyle en C1 à C4 ;
- R8 représente le groupe de formule -X-G-R9 dans laquelle X, G et R9 sont tels que définis ci-dessus ;
- m1 est un nombre entier allant de 1 à 998 ; et
- m2 est un nombre entier allant de 2 à 500.

Selon l'invention, le polyamide siliconé utilisé comme agent structurant, peut être un homopolymère, c'est-à-dire un polymère comportant plusieurs motifs identiques, en particulier des motifs selon les formules définies précédemment.

Selon l'invention, on peut aussi utiliser un polyamide siliconé constitué par un copolymère comportant plusieurs motifs selon la première formule précédente différents, c'est-à-dire un polymère dans lequel l'un au moins des R1, R2, R3, R4, X, G, Y, m et n est différent dans l'un des motifs. Le copolymère peut être aussi formé de plusieurs motifs selon la seconde formule précédente, dans lequel l'un au moins des R1, R3, R7, R8, m1 et m2 est différent dans l'un au moins des motifs.

On peut encore utiliser un copolymère comportant au moins un motif selon la première formule et au moins un motif selon la seconde formule, les motifs selon la première formule et les motifs selon la seconde formule pouvant être identiques ou différents les uns des autres.

Selon une variante de l'invention, on peut encore utiliser un polyamide siliconé de type copolymère comprenant de plus au moins un motif hydrocarboné comportant deux groupes capables d'établir des interactions hydrogènes choisis parmi les groupes ester, amide, sulfonamide, carbamate, thiocarbamate, urée, thiourée et leurs combinaisons. Ces copolymères peuvent être des copolymères blocs, des copolymères séquencés ou des copolymères greffés.

Selon une variante de réalisation, les groupes capables d'établir des interactions hydrogènes sont des groupes amides de formule -C(O)NH- et - HN-C(O)-. Dans ce cas, l'agent gélifiant peut être, par exemple, un polymère comprenant au moins un motif selon la troisième ou la quatrième formule suivante : 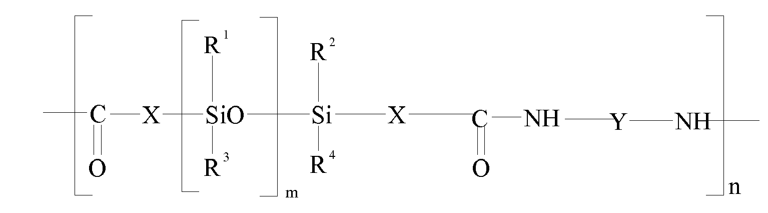 ou 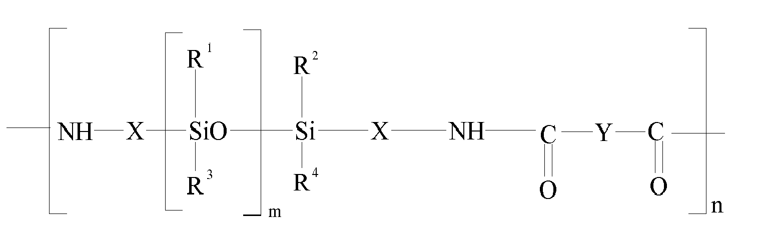 dans lesquelles R1, R2, R3, R4, X, Y, m et n sont tels que définis précédemment.

Dans les polyamides selon les troisième et quatrième formules précédemment exposées :
- m est notamment dans la gamme de 1 à 700, voire de 15 à 500 et mieux encore de 15 à 45, et
- n est en particulier dans la gamme de 1 à 500, notamment de 1 à 100 et mieux encore de 4 à 25,
- X est notamment une chaîne alkylène linéaire ou ramifiée ayant de 1 à 30 atomes de carbone, en particulier 3 à 10 atomes de carbone, et
- Y est notamment une chaîne alkylène linéaire ou ramifiée ou pouvant comporter des cycles et/ou des insaturations ayant de 1 à 40 atomes de carbone, en particulier de 1 à 20 atomes de carbone, et mieux encore de 2 à 6 atomes de carbone, en particulier 6 atomes de carbone.

Dans les troisième et quatrième formules exposées précédemment, le groupe alkylène représentant X ou Y peut éventuellement contenir dans sa partie alkylène au moins l'un des éléments suivants :
1) 1 à 5 groupes amides, urée ou carbamate,
2) un groupe cycloalkyle en C5 ou C6, et
3) un groupe phénylène éventuellement substitué par 1 à 3 groupes alkyles identiques ou différents en C1 à C3.

Dans les troisième et quatrième formules exposées précédemment, les groupes alkylènes peuvent aussi être substitués par au moins un élément choisi dans le groupe constitué de :
- un groupe hydroxy,
- un groupe cycloalkyle en C3 à C8,
- un à trois groupes alkyles en C1 à C40,
- un groupe phényle éventuellement substitué par un à trois groupes alkyles en C1 à C3,
- un groupe hydroxyalkyle en C1 à C3, et
- un groupe aminoalkyle en C1 à C6. Dans les troisième et quatrième formules exposées précédemment, Y peut aussi représenter : 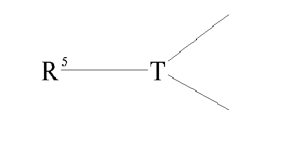
où R5 représente une chaîne polyorganosiloxane, et T représente un groupe de formule : 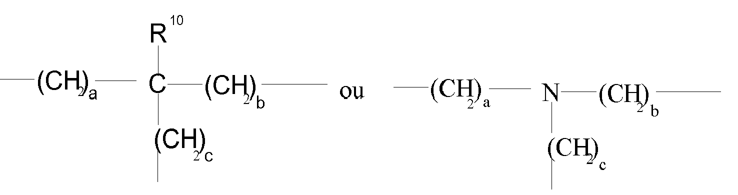 dans lesquelles a, b et c sont, indépendamment, des nombres entiers allant de 1 à 10, et R10 est un atome d'hydrogène ou un groupe tel que ceux définis pour R1, R2, R3, R4.

Dans les troisième et quatrième formules exposées précédemment, R1, R2, R3, R4 représentent notamment, indépendamment, un groupe alkyle en C1 à C40, linéaire ou ramifié, en particulier un groupe CH3, C2H5, n-C3H7 ou isopropyle, une chaîne polyorganosiloxane ou un groupe phényle éventuellement substitué par un à trois groupes méthyle ou éthyle.

Comme on l'a vu précédemment, le polymère peut également comprendre des motifs selon la troisième ou quatrième formule exposée précédemment identiques ou différents.

Ainsi, le polymère peut être un polyamide siliconé contenant plusieurs motifs selon la troisième ou quatrième formule exposée précédemment de longueurs différentes, soit un polyamide répondant à la cinquième formule suivante : 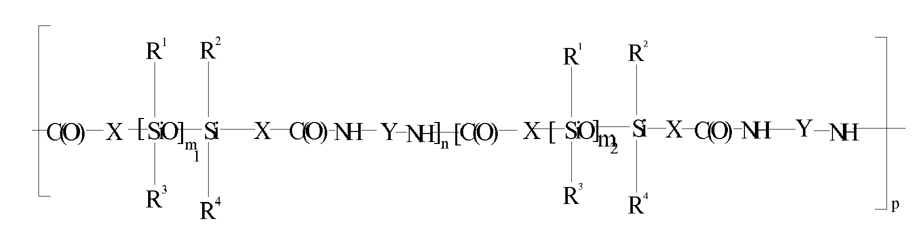 dans laquelle X, Y, n, R1 à R4 ont les significations données précédemment, m1 et m2 qui sont différents, sont choisis dans la gamme allant de 1 à 1000, et p est un nombre entier allant de 2 à 300.

Dans cette formule, les motifs peuvent être structurés pour former soit un copolymère bloc, soit un copolymère aléatoire, soit un copolymère alterné. Dans ce copolymère, les motifs peuvent être non seulement de longueurs différentes mais aussi de structures chimiques différentes, par exemple ayant des Y différents. Dans ce cas, le copolymère peut répondre à la sixième formule : 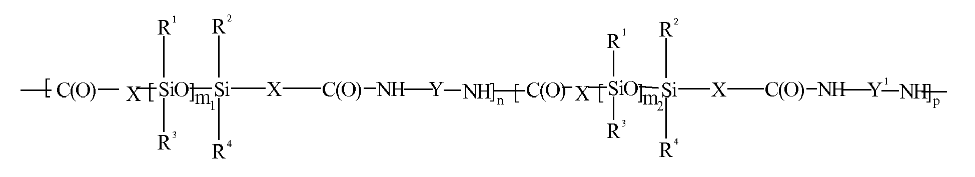 dans laquelle R1 à R4, X, Y, m1, m2, n et p ont les significations données ci-dessus et Y1 est différent de Y, mais est choisi parmi les groupes définis pour Y. Comme précédemment, les différents motifs peuvent être structurés pour former soit un copolymère bloc, soit un copolymère aléatoire, soit un copolymère alterné.

Selon un mode de réalisation de l'invention, le polyamide siliconé gélifiant peut être aussi constitué par un copolymère greffé. Ainsi, le polyamide à unités silicone peut être greffé et éventuellement réticulé par des chaînes silicones à groupes amides. De tels polymères peuvent être synthétisés avec des amines trifonctionnelles.

Dans ce cas, le copolymère peut comprendre au moins un motif selon la septième formule suivante : 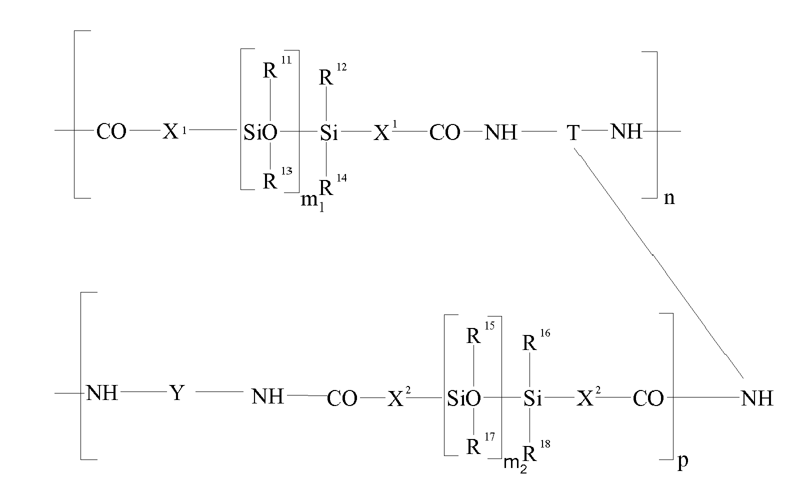 dans laquelle X1 et X2 qui sont identiques, ou différents, ont la signification donnée pour X dans la première formule précédente, n est tel que défini dans la première formule précédente, Y et T sont tels que définis dans la première formule précédente, R11 à R18 sont des groupes choisis dans le même groupe que les R1 à R4, m1 et m2 sont des nombres situés dans la gamme allant de 1 à 1 000, et p est un nombre entier allant de 2 à 500.
Dans la septième formule précédemment exposée, en particulier :
- p est dans la gamme de 1 à 25, mieux encore de 1 à 7,
- R11 à R18 sont des groupes méthyle,
- T répond à l'une des formules suivantes : 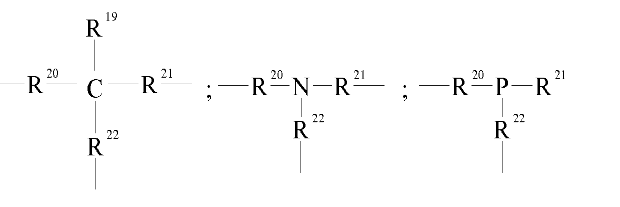 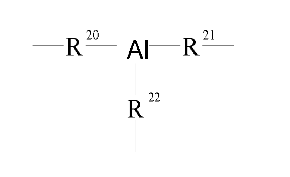
dans lesquelles R19 est un atome d'hydrogène ou un groupe choisi parmi les groupes définis pour R1 à R4, et R20, R21 et R22 sont, indépendamment, des groupes alkylène, linéaires ou ramifiés,

T répond en particulier de préférence à la formule : 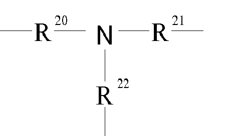 avec notamment R20, R21 et R22 représentant -CH2-CH2-,
- m1 et m2 sont dans la gamme de 15 à 500, voire de 15 à 45,
- X1 et X2 représentent -(CH2)10-, et
- Y représente -CH2-.

Ces polyamides à motif silicone greffé selon la septième formule exposée précédemment peuvent être copolymérisés avec des polyamides-silicones selon la seconde formule pour former des copolymères blocs, des copolymères alternés ou des copolymères aléatoires. Le pourcentage en poids de motifs siliconés greffés selon la septième formule dans le copolymère peut aller de 0,5 à 30 % en poids.

Selon un mode de réalisation, les unités siloxanes peuvent être dans la chaîne principale ou squelette du polymère, mais elles peuvent également être présentes dans des chaînes greffées ou pendantes. Dans la chaîne principale, les unités siloxanes peuvent être sous forme de segments comme décrits ci-dessus. Dans les chaînes pendantes ou greffées, les unités siloxanes peuvent apparaître individuellement ou en segments.

Selon un mode de réalisation de l'invention, les polyamides à base de siloxanes peuvent notamment être :
- les polyamides selon la troisième formule précédemment exposée où m est de 15 à 50 ;
- les mélanges de deux ou plusieurs polyamides dans lesquels au moins un polyamide a une valeur de m dans la gamme de 15 à 50 et au moins un polyamide a une valeur de m dans la gamme de 30 à 50 ; des polymères selon la cinquième formule décrite précédemment avec m1 choisi dans la gamme de 15 à 50 et m2 choisi dans la gamme de 30 à 500 avec la partie correspondant à m1 représentant 1 à 99 % en poids du poids total du polyamide et la partie correspondant à m2 représentant 1 à 99 % en poids du poids total du polyamide ;
- des mélanges de polyamide selon la troisième formule décrite précédemment combinant :
   1) 80 à 99 % en poids d'un polyamide où n est égal à 2 à 10, en particulier 3 à 6, et
   2) 1 à 20 % d'un polyamide où n est dans la gamme de 5 à 500, en particulier de 6 à 100 ;
- des polyamides répondant à la sixième formule précédemment exposée où au moins l'un des Y et Y1 contient au moins un substituant hydroxyle.
- des polyamides selon la troisième formule synthétisés avec au moins une partie d'un diacide activé (chlorure, dianhydride ou diester de diacide) au lieu du diacide ;
- des polyamides selon la troisième formule où X représente - (CH2)3- ou -(CH2)10 ; et
- des polyamides selon la troisième formule où les polyamides sont terminés par une chaîne monofonctionnelle choisie dans le groupe constitué des amines monofonctionnelles, des acides monofonctionnels, des alcools monofonctionnels, incluant les acides gras, les alcools gras et les amines grasses, tels que par exemple l'octylamine, l'octanol, l'acide stéarique et l'alcool stéarylique.

Selon un mode de réalisation de l'invention, les extrémités des chaînes du polymère peuvent être terminées par :
- un groupe ester d'alkyle en C1 à C50 en introduisant en cours de synthèse un monoalcool en C1 en C50,
- un groupe amide d'alkyle en C1 à C50 en prenant comme stoppeur un monoacide si la silicone est alpha , omega -diaminée, ou une monoamine si la silicone est alpha , omega -diacide carboxylique.

Selon une autre variante de réalisation de l'invention, on peut utiliser un copolymère de polyamide silicone et de polyamide hydrocarboné, soit un copolymère comportant des motifs selon la troisième ou la quatrième formule et des motifs polyamide hydrocarboné. Dans ce cas, les motifs polyamide-silicone peuvent être disposés aux extrémités du polyamide hydrocarboné.

Des agents gélifiants à base de polyamide contenant des silicones peuvent être produits par amidation silylique de polyamides à base de dimère d'acide gras. Cette approche implique la réaction de sites acides libres existant sur un polyamide comme sites terminaux, avec des oligosiloxanes-monoamines et/ou des oligosiloxanes-diamines (réaction d'amidation), ou alternativement avec des oligosiloxanes alcools ou des oligosiloxanes diols (réaction d'estérification). La réaction d'estérification nécessite la présence de catalyseurs acides, comme il est connu dans la technique. Il est souhaitable que le polyamide ayant des sites acides libres, utilisés pour la réaction d'amidation ou d'estérification, ait un nombre relativement élevé de terminaisons acides (par exemple des polyamides ayant des indices d'acide élevés, par exemple de 15 à 20).

Pour l'amidation des sites acides libres des polyamides hydrocarbonés, des siloxanes diamines avec 1 à 300, plus particulièrement 2 à 50, et mieux encore 2, 6, 9, 5, 12, 13, 5, 23 ou 31 groupes siloxanes, peuvent être utilisés pour la réaction avec des polyamides hydrocarbonés à base de dimères d'acide gras. On préfère des siloxanes diamines ayant 13,5 groupes siloxanes et les meilleurs résultats sont obtenus avec la siloxane-diamine ayant 13,5 groupes siloxane et des polyamides contenant des indices élevés de groupes terminaux acides carboxyliques.

Les réactions peuvent être effectuées dans le xylène pour extraire l'eau produite de la solution par distillation azéotropique, ou à des températures plus élevées (autour de 180 à 200 °C) sans solvant. Typiquement, l'efficacité de l'amidation et les taux de réaction diminuent lorsque le siloxane diamine est plus long, c'est-à-dire lorsque le nombre de groupes siloxanes est plus élevé. Des sites amines libres peuvent être bloqués après la réaction d'amidation initiale des diaminosiloxanes en les faisant réagir avec soit un siloxane acide, soit un acide organique tel que l'acide benzoïque.

Pour l'estérification des sites acides libres sur les polyamides, ceci peut être réalisé dans le xylène bouillant avec environ 1 % en poids, par rapport au poids total des réactifs, d'acide paratoluènesulfonique comme catalyseur.

Ces réactions effectuées sur les groupes acides carboxyliques terminaux du polyamide conduisent à l'incorporation de motifs silicone seulement aux extrémités de la chaîne de polymère.

A titre d'exemple on citera le DC2- 8178 Gellant et le DC2-8179 Gellant de Dow Corning.

### 4) Les mono- ou polyalkylesters de saccharide ou polysaccharide

Parmi les mono ou polyalkylesters de saccharide ou de polysaccharide convenant à la mise en oeuvre de l'invention, on peut mentionner les alkyles ou polyalkylesters de dextrine ou d'inuline.

Il peut s'agir notamment d'un mono-ou poly-ester de dextrine et d'au moins un acide gras et notamment répondant à la formule suivante : 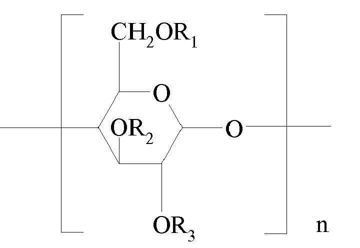 dans laquelle :
- n est un entier allant de 3 à 200, notamment allant de 20 à 150, et en particulier allant de 25 à 50,
- les radicaux R1, R2 et R3, identiques ou différents, sont choisis parmi l'hydrogène ou un groupement acyle (R-CO-) dans lequel le radical R est un groupement hydrocarboné, linéaire ou ramifié, saturé ou insaturé, possédant de 7 à 29, en particulier de 7 à 21, notamment de 11 à 19, plus particulièrement de 13 à 17, voire 15, atomes de carbone, sous réserve qu'au moins un desdits radicaux R1, R2 ou R3 est différent de l'hydrogène.

En particulier, R1, R2 et R3 peuvent représenter l'hydrogène ou un groupement acyle (R-CO-) dans lequel R est un radical hydrocarboné tel que défini précédemment, sous réserve qu'au moins deux desdits radicaux R1, R2 ou R3 sont identiques et différents de l'hydrogène.

L'ensemble des radicaux R1, R2 et R3 peut figurer un groupement acyle (R-CO) identique ou différent, et notamment identique.

En particulier, n précédemment exposé varie avantageusement de 25 à 50, notamment est égal à 38 dans la formule générale de l'ester de saccharide utilisable dans la présente invention.

Notamment lorsque les radicaux R1, R2 et/ou R3, identiques ou différents figurent un groupement acyle (R-CO), ceux-ci peuvent être choisis parmi les radicaux caprylique, caprique, laurique, myristique, palmitique, stéarique, arachique, béhénique, isobutyrique, isovalérique, éthyl-2 butyrique, éthylméthylacétique, isoheptanoïque, éthyl-2 hexanoïque, isononanoïque, isodécanoïque, isotridécanoïque, isomyristique, isopalmitique, isostéarique, isoaracique, isohexanoïque, décénoïque, dodécenoïque, tetradécénoïque, myristoléïque, hexadécénoïque, palmitoléïque, oléïque, élaidique, asclépinique, gondoléïque, eicosènoïque, sorbique, linoléïque, linolénique, punicique, stéaridonique, arachidonique, stéarolique, et leurs mélanges.

De préférence, on utilise à titre d'ester de dextrine et d'acide(s) gras au moins un palmitate de dextrine. Celui-ci peut être utilisé seul ou en mélange avec d'autres esters.

Avantageusement, l'ester de dextrine et d'acide gras a un degré de substitution inférieur ou égal à 2,5 sur la base d'une unité glucose, notamment variant de 1,5 à 2,5, de préférence de 2 à 2,5. Le poids moléculaire moyen en poids de l'ester de dextrine peut être en particulier de 10 000 à 150 000, notamment de 12 000 à 100 000 et voire de 15 000 à 80 000.

Des esters de dextrine, en particulier des palmitates de dextrine, sont disponibles commercialement sous la dénomination RHEOPEARL TL ou RHEOPEARL KL de la société Chiba Flour.

### 5) les dérivés amides d'aminoacides N-acylés

Les amides d'aminoacides N-acylés utilisables sont par exemple les diamides de l'association d'un acide N-acylamine avec des amines comprenant de 1 à 22 atomes de carbone tels que ceux décrits dans le document FR 2 281 162. Ce sont par exemple les dérivés amides d'acide alcoyle glutamique tels que le dibutylamide d'acide laurylglutamique, commercialisé par la société Ajinomoto sous le nom « Gelling agent GP-1 » ou encore le dibutylamide d'acide 2-éthylhexanoyl glutamique commercialisé par la société Ajinomoto sous la dénomination de « Gelling agent GA-01 ».

### 6) Les copolymères comprenant une séquence alkylène ou styrène

Les copolymères peuvent avoir une structure en peigne ou bloc de type di-bloc, tri-bloc, multi-bloc et/ou radial ou étoilé et comporter au moins deux segments incompatibles du point de vue thermodynamique.

L'agent structurant peut comprendre, par exemple, un bloc segment styrène comme décrit dans les demandes EP 0 497 144, WO98/42298, US 6 225 690, US 6 174 968, US 6 225 390, un segment éthylène/butylène, un segment éthylène/propylène comme décrit dans les demandes US 6 225 690, US 6 174 968, US 6 225 390, un segment butadiène, un segment isoprène, un segment polyvinyl comme par exemple poly(meth)acrylate d'alkyle,ou polyvinyl alcool ou polyacétate de vinyl, un segment siliconé comme décrit dans les demandes US 5 468 477 et US 5 725 882 ou une combinaison de ces segments.

Un copolymère di-bloc est habituellement défini comme étant de type A-B dans lequel un segment dur (A) est suivi par un segment souple (B).

Un copolymère tri-bloc est habituellement défini comme étant de type A-B-A ou comme un rapport d'un segment dur, d'un segment souple et d'un segment dur.

Un copolymère multi-bloc ou radial ou étoilé peut comporter n'importe quel type de combinaison de segments durs et de segments souples, sous réserve que les caractéristiques des segments durs et des segments souples soient conservées.

A titre d'exemple de segments durs de copolymère bloc, il peut être fait mention du styrène, et à titre d'exemple de segments souples de copolymère bloc, il peut être fait mention de l'éthylène, du propylène, du butylène, et d'une combinaison de ceux-ci.

Les copolymères tri-bloc, et notamment ceux de type polystyrène/polyisoprène ou polystyrène/polybutadiène, convenant à la mise en oeuvre de l'invention peuvent être ceux commercialisés sous la référence LUVITOL HSB par la société BASF. Il peut également être fait mention des copolymères tri-bloc de type polystyrène/copoly(éthylènepropylène) ou polystyrène/ copoly(éthylène -butylène), tels que ceux commercialisés sous la référence KRATON par la société SHELL CHEMICAL CO, ou sous la référence GELLED PERMETHYL 99 A par la société PENRECO. De tels copolymères tri-blocs sont particulièrement préférés selon l'invention.

A titre d'exemple, encore, de copolymères bloc susceptibles de convenir à la mise en oeuvre de la présente invention, il peut également être fait mention des copolymères blocs commercialisés sous la référence VERSAGEL par la société PENRECO, ceux commercialisés sous la référence KRATON par la société SHELL et ceux commercialisés sous la référence GEL BASE par la société BROOKS INDUSTRIES.

Parmi les polymères épaississants pour phase grasse les polymères portant dans le squelette au moins une séquence cristallisable sont préférés.

Les polymères épaississants pour phase aqueuse ou phase grasse peuvent être utilisés seuls ou en mélanges en toutes proportions.

De préférence le ou les polymères épaississants sont choisis parmi les polymères épaississants de phase aqueuse.

Le ou les polymères épaississants est ou sont présents dans la composition selon l'invention dans une teneur allant de 0,01 à 10 % en poids et de préférence de 0,1 à 5 % en poids par rapport au poids total de la composition.

La composition selon l'invention comprend un ou plusieurs précurseurs de colorants.

Ce ou ces précurseurs de colorants sont choisis parmi les bases d'oxydation et les coupleurs.

La ou les bases d'oxydation utilisables dans le cadre de la présente invention sont choisies parmi celles classiquement connues en teinture d'oxydation, et parmi lesquelles on peut notamment citer les ortho- et para-phénylènediamines, les bases doubles, les ortho- et para-aminophénols, les bases hétérocycliques ainsi que les sels d'addition de ces composés avec un acide.

Ces bases d'oxydation peuvent être en particulier cationiques.

Les para-phénylènediamines utilisables dans le cadre de l'invention peuvent notamment être choisies parmi les composés de formule (XX) suivante et leurs sels d'addition avec un acide : 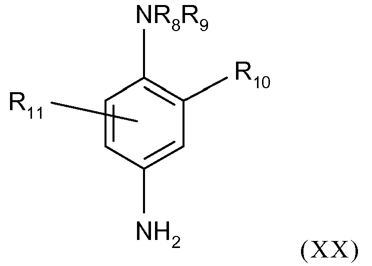 dans laquelle :
■ R8 représente un atome d'hydrogène, un radical alkyle en C1-C4, monohydroxyalkyle en C1-C4, polyhydroxyalkyle en C2-C4, alcoxy(C1-C4)alkyle(C1-C4), alkyle en C1-C4 substitué par un groupement azoté, phényle ou 4'-aminophényle ;
■ R9 représente un atome d'hydrogène, un radical alkyle en C1-C4, monohydroxyalkyle en C1-C4, polyhydroxyalkyle en C2-C4, alcoxy(C1-C4)alkyle(C1-C4) ou alkyle en C1-C4 substitué par un groupement azoté ;
■ R8 et R9 peuvent également former avec l'atome d'azote qui les porte un hétérocycle azoté à 5 ou 6 chaînons éventuellement substitué par un ou plusieurs groupements alkyle, hydroxy ou uréido ;
■ R₁₀ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄ ;
■ R₁₁ représente un atome d'hydrogène, d'halogène ou un radical alkyle en C₁-C₄.

Parmi les groupements azotés de la formule (XX) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les para-phénylènediamines de formule (XX) ci-dessus, on peut plus particulièrement citer la para-phénylènediamine, la para-toluylènediamine, la 2-chloro-para-phénylènediamine, la 2,3-diméthyl-para-phénylènediamine, la 2,6-diméthyl-para-phénylènediamine, la 2,6-diéthyl-para-phénylènediamine, la 2,5-diméthyl-para-phénylènediamine, la N,N-diméthyl-para-phénylènediamine, la N,N-diéthyl-para-phénylènediamine, la N,N-dipropyl-para-phénylènediamine, la 4-amino-N,N-diéthyl-3-méthyl-aniline, la 4-N,N-bis-(β-hydroxyéthyl)amino-2-méthyl-aniline, la N,N bis β-hydroxyéthyl paraphénylènediamine, la 4-N,N-bis-(β-hydroxyéthyl)-amino-2-chloro-aniline, la 2-β-hydroxyéthyl-para-phénylènediamine, la 2-fluoro-para-phénylènediamine, la 2-isopropyl-para-phénylènediamine, la N-(β-hydroxypropyl)-para-phénylènediamine, la 2-hydroxyméthyl-para-phénylènediamine, la N,N-diméthyl-3-méthyl-para-phénylènediamine, la N,N-(éthyl,β-hydroxyéthyl)-para-phénylènediamine, la N-(β,γ-dihydroxypropyl)-para-phénylènediamine, la N-(4'-aminophényl)-para-phénylènediamine, la N-phényl-para-phénylènediamine, la 2-β-hydroxyéthyloxy-para-phénylènediamine, la 2-β-acétylaminoéthyloxy-para-phénylènediamine, la N-(β-méthoxyéthyl)-para-phénylènediamine, la 2-méthyl-1-N-β-hydroxyéthyl-para-phénylènediamine, et leurs sels d'addition avec un acide.

Parmi les para-phénylènediamines de formule (XX) ci-dessus, on préfère tout particulièrement la para-phénylènediamine, la para-toluylènediamine, la 2-isopropyl-para-phénylènediamine, la 2-β-hydroxyéthyl-para-phénylènediamine, la 2,6-diméthyl-para-phénylène-diamine, la 2,6-diéthyl-para-phénylènediamine, la 2,3-diméthyl-para-phénylènediamine, la 2-chloro-para-phénylènediamine, la N,N bis β-hydroxyéthyl paraphénylènediamine et leurs sels d'addition avec un acide.

On utilisera tout particulièrement la para-phénylènediamine et la para-toluylènediamine, la N,N bis β-hydroxyéthyl paraphénylènediamine et leurs sels d'addition avec un acide.

Selon l'invention, on entend par bases doubles les composés comportant au moins deux noyaux aromatiques sur lesquels sont portés des groupements amino et/ou hydroxyle.

Parmi les bases doubles utilisables à titre de bases d'oxydation dans la composition conforme à l'invention, on peut notamment citer les composés répondant à la formule (XXI) suivante et leurs sels d'addition avec un acide : 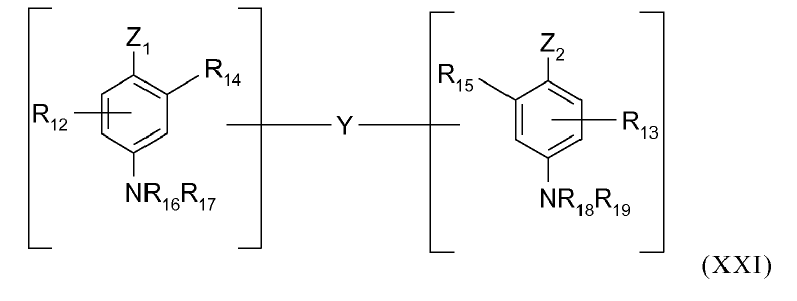 dans laquelle :
■ Z₁ et Z₂, identiques ou différents, représentent un radical hydroxyle ou -NH₂ pouvant être substitué par un radical alkyle en C₁-C₄ ou par un bras de liaison Y ;
■ le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
■ R₁₂ et R₁₃ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y;
■ R₁₄, R₁₅, R₁₆, R₁₇, R₁₈ et R₁₉, identiques ou différents, représentent un atome d'hydrogène, un bras de liaison Y ou un radical alkyle en C₁-C₄ ;
■ étant entendu que les composés de formule (XXI) ne comportent qu'un seul bras de liaison Y par molécule.

Parmi les groupements azotés de la formule (XXI) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formules (XXI) ci-dessus, on peut plus particulièrement citer le N,N'-bis-(β-hydroxyéthyl)-N,N'-bis-(4'-aminophényl)-1,3-diamino-propanol, la N,N'-bis-(β-hydroxyéthyl)-N,N'-bis-(4'-aminophényl)-éthylènediamine, la N,N'-bis-(4-aminophényl)-tétraméthylènediamine, la N,N'-bis-(β-hydroxyéthyl)-N,N'-bis-(4-aminophényl)-tétraméthylènediamine, la N,N'-bis-(4-méthyl-aminophényl)-tétraméthylènediamine, la N,N'-bis-(éthyl)-N,N'-bis-(4'-amino-3'-méthylphényl)-éthylènediamine, le 1,8-bis-(2,5-diaminophénoxy)-3,5-dioxaoctane, et leurs sels d'addition avec un acide.

Parmi ces bases doubles de formule (XXI), le N,N'-bis-(β-hydroxyéthyl)-N,N'-bis-(4'-aminophényl)-1,3-diamino-propanol, le 1,8-bis-(2,5-diaminophénoxy)-3,5-dioxaoctane ou l'un de leurs sels d'addition avec un acide sont particulièrement préférés.

Les para-aminophénols utilisables dans le cadre de l'invention peuvent notamment être choisis parmi les composés répondant à la formule (XXII) suivante et leurs sels d'addition avec un acide : 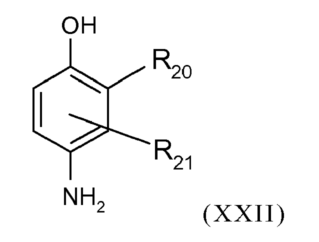 dans laquelle :
■ R₂₀ représente un atome d'hydrogène, un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄ ;
■ R₂₁ représente un atome d'hydrogène ou un atome d'halogène tel que le fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).

Parmi les para-aminophénols de formule (XXII) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.

Le para-aminophénol et le 4-amino-3-méthyl-phénol sont encore plus préférés.

Les ortho-aminophénols utilisables à titre de bases d'oxydation dans le cadre de la présente l'invention sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.

Parmi les bases hétérocycliques utilisables à titre de bases d'oxydation dans la composition conforme à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, la 3,4-diamino-pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl-pyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol; le 2-(7-amino-pyrazolo-[1,5-a]-pyrimidin-3-ylamino)-éthanol ; le 2-[(3-amino-pyrazolo[1,5-a]pyrimidin-7-yl)-(2-hydroxy-éthyl)-amino]-éthanol ; le 2-[(7-amino-pyrazolo[1,5-a]pyrimidin-3-yl)-(2-hydroxy-éthyl)-amino]-éthanol ; la 5,6-diméthyl-pyrazolo-[1,5-a]-pyrimidine-3,7-diamine ; la 2,6-diméthyl-pyrazolo-[1,5-a]-pyrimidine-3,7-diamine ; la 2,5,N7,N7-tétraméthyl-pyrazolo-[1,5-a]-pyrimidine-3,7-diamine ; la 3-amino-5-méthyl-7-imidazolylpropylamino-pyrazolo-[1,5-a]-pyrimidine ; leurs sels d'addition et leurs formes tautomères, lorsqu'il existe un équilibre tautomérique.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme les 4,5-diaminopyrazoles tels que par exemple le 4,5-diamino-1-méthyl-pyrazole, le 4,5-diamino-1-(4'-chlorobenzyl)-pyrazole, le 4,5-diamino-1,3-diméthyl-pyrazole, le 4,5-diamino-3-méthyl-1-phényl-pyrazole, le 4,5-diamino-1-méthyl-3-phényl-pyrazole, le 1-benzyl-4,5-diamino-3-méthyl-pyrazole, le 4,5-diamino-3-tert-butyl-1-méthyl-pyrazole, le 4,5-diamino-1-tert-butyl-3-méthyl-pyrazole, le 4,5-diamino-1-(β-hydroxyéthyl)-3-méthyl-pyrazole, le 4,5-diamino-1-(β-hydroxyéthyl)-pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole et le 4-amino-5-(2'-aminoéthyl)amino-1,3-diméthyl-pyrazole ; le 3,4-diamino-pyrazole ; le 4-amino-1,3-diméthyl-5-hydrazino-pyrazole ; les 3,4,5-triaminopyrazoles tels que par exemple le 3,4,5-triamino-pyrazole, le 1-méthyl-3,4,5-triamino-pyrazole, le 3,5-diamino-1-méthyl-4-méthylamino-pyrazole, le 3,5-diamino-4-(β-hydroxyéthyl)amino-1-méthyl-pyrazole ; et leurs sels d'addition avec un acide.

De préférence, on utilisera un 4,5-diaminopyrazole et encore plus préférentiellement le 4,5-diamino-1-(β-hydroxyéthyl)-pyrazole et/ou l'un des ses sels.

A titre de dérivés pyrazoliques, on peut également citer les diamino N,N-dihydropyrazolopyrazolones et notamment celles décrites dans la demande FR-A-2 886 136 telles que les composés suivants et leurs sels d'addition : 2,3-diamino-6,7-dihydro-1H,5H-pyrazolo[1,2-a]pyrazol-1-one, 2-amino-3-éthylamino-6,7-dihydro-1H,5H-pyrazolo[1,2-a]pyrazol-1-one, 2-amino-3-isopropylamino-6,7-dihydro-1H,5H-pyrazolo[1,2-a]pyrazol-1-one, 2-amino-3-(pyrrolidin-1-yl)-6,7-dihydro-1H,5H-pyrazolo[1,2-a]pyrazol-1-one, 4,5-diamino-1,2-diméthyl-1,2-dihydro-pyrazol-3-one, 4,5-diamino-1,2-diéthyl-1,2-dihydro-pyrazol-3-one, 4,5-diamino-1,2-di-(2-hydroxyéthyl)-1,2-dihydro-pyrazol-3-one, 2-amino-3-(2-hydroxyéthyl)amino-6,7-dihydro-1H,5H-pyrazolo[1,2-a]pyrazol-1-one, 2-amino-3-diméthylamino-6,7-dihydro-1H,5H-pyrazolo[1,2-a]pyrazol-1-one, 2,3-diamino-5,6,7,8-tétrahydro-1H,6H-pyridazino[1,2-a]pyrazol-1-one, 4-amino-1,2-diéthyl-5-(pyrrolidin-1-yl)-1,2-dihydro-pyrazol-3-one, 4-amino-5-(3-diméthylamino-pyrrolidin-1-yl)-1,2-diéthyl-1,2-dihydro-pyrazol-3-one, 2,3-diamino-6-hydroxy-6,7-dihydro-1H,5H-pyrazolo[1,2-a]pyrazol-1-one.

On préférera utiliser la 2,3-diamino-6,7-dihydro-1H,5H-pyrazolo[1,2-a]pyrazol-1-one et/ou un de ses sels d'addition.

A titre de bases hétérocycliques, on utilisera préférentiellement le 4,5-diamino-1-(β-hydroxyéthyl)pyrazole et/ou la 2,3-diamino-6,7-dihydro-1H,5H-pyrazolo[1,2-a]pyrazol-1-one et/ou un de leurs sels d'addition.

A titre de bases d'oxydation cationiques utilisables dans les compositions selon l'invention, on peut citer par exemple les composés suivants : les para-phénylènediamines telles que notamment décrites dans les demandes de brevets FR-A-2 766 177 et FR-A-2 766 178, les para-aminophénols tels que décrits par exemple dans les demandes de brevet FR-A-2 766 177 et FR-A-2 766 178, les ortho-phénylènediamines telles que décrites par exemple dans les demandes de brevet FR-A-2 782 718, FR-A-2 782 716 et FR-A-2 782 719, des ortho-aminophénols ou des bases doubles cationiques telles que des dérivés de type bis(aminophényl)alkylènediamine décrites dans les demandes de brevet FR-A-2 766 179, ainsi que les bases hétérocycliques cationiques, ces composés portant au moins un atome d'azote quaternaire.

De préférence, les bases d'oxydation cationiques utilisables dans les compositions selon l'invention sont des para-phénylènediamines cationiques.

De manière avantageuse, une variante consiste à mettre en oeuvre des bases d'oxydation cationiques de structure para-phénylènediamine, dont au moins une des fonctions amine est une amine tertiaire porteuse d'un noyau pyrrolidinique, la molécule possédant au moins un atome d'azote quaternisé. De telles bases sont, par exemple, décrites dans le document EP-A-1 348 695.

La composition selon l'invention comprend de préférence une quantité totale de bases d'oxydation allant de 0,0005 à 12 % en poids par rapport au poids total de la composition. De préférence, elle comprend une quantité totale de bases d'oxydation allant de 0,005 à 8 % en poids, et mieux encore de 0,05 à 5 % en poids, par rapport au poids total de ladite composition.

Le ou les coupleurs utilisables dans la composition selon l'invention sont ceux classiquement utilisés dans les compositions de teinture d'oxydation, c'est-à-dire les méta-aminophénols, les méta-phénylènediamines, les métadiphénols, les naphtols et les coupleurs hétérocycliques tels que, par exemple, les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines, et les sels d'addition de ces composés avec un acide.

Ces coupleurs sont plus particulièrement choisis parmi le 2,4-diamino 1-(β-hydroxyéthyloxy)-benzène, le 2-méthyl-5-amino-phénol, le 5-N-(β-hydroxyéthyl)amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2-amino 4-(β-hydroxyéthylamino)-1-méthoxy-benzène, le 1,3-diamino-benzène, le 1,3-bis-(2,4-diaminophénoxy)-propane, le sésamol, le 1-amino-2-méthoxy-4,5-méthylènedioxy benzène, l'α-naphtol, le 6-hydroxy-indole, le 4-hydroxy-indole, le 4-hydroxy-N-méthyl indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, la 2-amino-3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

La composition selon l'invention comprend généralement une quantité totale de coupleurs allant de 0,0001 à 15 % en poids par rapport au poids total de la composition. De préférence, elle comprend une quantité totale de coupleurs allant de 0,001 à 10 % en poids, et mieux encore de 0,01 à 8 % en poids, par rapport au poids total de la composition.

Les bases d'oxydation et coupleurs peuvent être présents dans les compositions de l'invention, sous forme de sels d'addition, et en particulier sous forme de sels d'addition avec un acide.

Les sels d'addition avec un acide utilisables dans le cadre de l'invention sont, notamment, choisis parmi les chlorhydrates, les bromhydrates, les sulfates, les citrates, les succinates, les tartrates, les lactates, les acétates, les alkylsulfates et les alkylsulfonates.

Lorsque les bases d'oxydation ou les coupleurs contiennent une ou plusieurs fonctions acide carboxylique ou sulfonique, des sels d'addition avec une base sont envisageables. Les sels d'addition avec une base utilisables dans le cadre des compositions tinctoriales de l'invention sont alors notamment ceux obtenus avec de la soude, de la potasse, de l'ammoniaque ou des amines.

Selon un mode de réalisation particulier de l'invention, la composition comprend une ou plusieurs bases d'oxydation et un ou plusieurs coupleurs.

Selon une variante, la base d'oxydation additionnelle est choisie parmi les para-aminophénols, les bases hétérocycliques ainsi que les sels d'addition avec un acide correspondant.

La composition conforme à la présente invention comprend un ou plusieurs agents oxydants.

Un tel agent oxydant est choisi par exemple parmi les peroxydes tels que le peroxyde d'hydrogène, le peroxyde d'urée, les bromates ou ferricyanures de métaux alcalins, les persels tels que les perborates, percarbonates et les persulfates. On peut également utiliser à titre d'agent oxydant une ou plusieurs enzymes d'oxydo-réduction telles que les laccases, les peroxydases et les oxydoréductases à 2 électrons (telles que l'uricase), éventuellement en présence de leur donneur ou cofacteur respectif.

L'utilisation du peroxyde d'hydrogène est particulièrement préférée. Cet agent oxydant est avantageusement constitué par une solution d'eau oxygénée dont le titre peut varier, plus particulièrement, d'environ 1 à 40 volumes (soit d'environ 0.3 à 12% de peroxyde d'hydrogène), et encore plus préférentiellement d'environ 5 à 40 volumes (soit d'environ 1.5 à 12% de peroxyde d'hydrogène).

La concentration en agent(s) oxydant(s) de la composition de l'invention va de préférence de 0.1 à 20% mieux de 0.5 à 10% du poids total de la composition.

La composition de l'invention comprend un ou plusieurs agents alcalins dont au moins un de ces agents est une amine organique. Ce ou ces agents alcalins sont par exemple choisis parmi l'ammoniaque, les carbonates ou bicarbonates alcalins et en particulier de sodium ou de potassium, les alcanolamines telles que les mono-, di- et triéthanolamines ainsi que leurs dérivés, les hydroxyalkylamines et les éthylènediamines oxyéthylénées et/ou oxypropylénées, les hydroxydes de sodium ou de potassium, les acides aminés et en particulier les acides aminés basiques comme l'arginine ou la lysine et les composés de formule (XXIII) suivante : 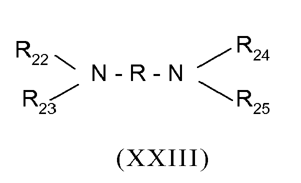 dans laquelle :
■ R est un reste propylène éventuellement substitué par un groupement hydroxyle ou un radical alkyle en C₁-C₄ ;
■ R₂₂, R₂₃, R₂₄ et R₂₅, identiques ou différents, représentent un atome d'hydrogène, un radical alkyle en C₁-C₄ ou hydroxyalkyle en C₁-C₄.

Selon un mode de réalisation particulier, la composition contient en tant qu'agents alcalins au moins une amine organique choisi parmi les alkanolamine. Lorsque la composition contient plusieurs agents alcalins dont une alkanolamine et de l'ammoniaque ou un de ses sels, la ou les amines organiques sont de préférence majoritaire en poids par rapport à la quantité ammoniac.

Selon un autre mode de réalisation particulier, la composition contient une faible quantité d'ammoniaque, voire pas d'ammoniaque. Selon ce mode de réalisation, la composition contient de préférence une ou plusieurs alcanolamines, notamment la monoéthanolamine.

La concentration en agent(s) alcalin(s) de la composition de l'invention va de préférence de 0.01 à 30%, et encore plus préférentiellement de 0.1 à 20% du poids total de la composition.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques, et leurs sels d'addition. Ces colorants directs peuvent être de nature non- ionique, anionique ou cationique.

La composition peut aussi contenir d'autres composés constituant le milieu de coloration. Ce milieu de coloration comprend généralement de l'eau ou un mélange d'eau et d'un ou plusieurs solvant(s) organique(s) acceptable(s), de préférence hydrosolubles sur le plan cosmétique.

A titre d'exemples de solvants organiques, on peut notamment citer les alcools tels que l'alcool éthylique, l'alcool isopropylique, l'alcool benzylique, et l'alcool phényléthylique, ou les glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, l'hexylène glycol, le dipropylèneglycol ainsi que les alkyléthers de diéthylèneglycol comme par exemple, le monoéthyléther ou le monobutyléther du diéthylèneglycol. Les solvants peuvent alors être présents dans des concentrations comprises entre environ 0,01 à 35 % en poids et, de préférence, entre environ 0,1 et 25 % en poids par rapport au poids total de la composition.

De préférence la composition de l'invention contient de l'eau. Encore plus préférentiellement la concentration en eau peut aller de 10 à 70%, mieux de 20 à 55% du poids total de la composition.

La composition conforme à l'invention peut contenir en outre un ou plusieurs adjuvant(s) utilisé(s) classiquement dans les compositions pour la teinture des cheveux.

Par "adjuvant", on entend un additif, différent des composés précités.

A titre d'exemples d'adjuvants utilisables, on peut citer les agents tensio-actifs anioniques, cationiques, non-ioniques, amphotères, zwittérioniques ou leurs mélanges ; les polymères anioniques, cationiques, non-ioniques, amphotères, zwittérioniques, les agents épaississants minéraux ou organiques, non polymériques; les agents antioxydants ou réducteurs ; les agents de pénétration ; les agents séquestrants ; les parfums ; les tampons ; les agents dispersants ; les agents conditionneurs tels que par exemple les silicones volatiles ou non volatiles, modifiées ou non modifiées ; les agents filmogènes ; les céramides ; les agents conservateurs ; les agents opacifiants ; et les agents anti-statique.

Les adjuvants ci-dessus sont en général présents en quantité comprise pour chacun d'eux entre 0,01 et 20 % en poids par rapport au poids de la composition tinctoriale.

De préférence la composition de l'invention contient un ou plusieurs tensioactifs.

De préférence, le ou les tensioactifs sont choisis parmi les tensioactifs non ioniques ou parmi les tensioactifs anioniques.

Les tensioactifs anioniques sont plus spécialement choisis parmi les sels (en particulier sels de métaux alcalins, notamment de sodium, sels d'ammonium, sels d'amines tels que les sels d'aminoalcools ou sels de métaux alcalino-terreux comme le magnésium) des composés suivants :
- les alkylsulfates, les alkyléthersulfates, alkylamidoéthersulfates, alkylaryl-polyéthersulfates, monoglycérides sulfates ;
- les alkylsulfonates, alkylamidesulfonates, alkylarylsulfonates, α - oléfine-sulfonates, paraffine-sulfonates ;
- les alkylphosphates, les alkylétherphosphates;
- les alkylsulfosuccinates, les alkyléthersulfosuccinates, les alkylamide-sulfosuccinates; les alkylsulfosuccinamates ;
- les alkylsulfoacétates ;
- les acylsarcosinates ; les acyliséthionates et les N-acyltaurates ;
- les sels d'acides gras tels que les acides oléique, ricinoléique, palmitique, stéarique, les acides d'huile de coprah ou d'huile de coprah hydrogénée ;
- les sels d'acides d'alkyl D galactoside uroniques ;
- les acyl-lactylates ;
- les sels des acides alkyléther carboxyliques polyoxyalkylénés, des acides alkylaryléther carboxyliques polyoxyalkylénés, des acides alkylamidoéther carboxyliques polyoxyalkylénés, en particulier ceux comportant de 2 à 50 groupements oxyde d'éthylène ;
- et leurs mélanges.

Il est à noter que le radical alkyle ou acyle de ces différents composés comporte avantageusement de 6 à 24 atomes de carbone, et de préférence de 8 à 24 atomes de carbone, et le radical aryle désignant de préférence un groupement phényle ou benzyle.

Les tensioactifs non ioniques sont plus particulièrement choisis parmi les tensioactifs non ioniques mono ou poly- oxyalkylénés, mono- ou poly-glycérolés. Les motifs oxyalkylénés sont plus particulièrement des motifs oxyéthylénés, oxypropylénés, ou leur combinaison, de préférence oxyéthylénés.

A titre d'exemples de tensioactifs non ioniques oxyalkylénés, on peut citer :
- les alkyl(C₈-C₂₄)phénols oxyalkylénés,
- les alcools en C₈-C₃₀, saturés ou non, linéaires ou ramifiés, oxyalkylénés,
- les amides, en C₈-C₃₀, saturés ou non, linéaires ou ramifiés, oxyalkylénés,
- les esters d'acides en C₈-C₃₀, saturés ou non, linéaires ou ramifiés, et de polyéthylèneglycols,
- les esters d'acides en C₈-C₃₀, saturés ou non, linéaires ou ramifiés, et de sorbitol polyoxyéthylénés,
- les huiles végétales oxyéthylénées, saturées ou non,
- les condensats d'oxyde d'éthylène et/ou d'oxyde de propylène, entre autres, seuls ou en mélanges.

Les tensioactifs présentent un nombre de moles d'oxyde d'éthylène et/ou de propylène compris entre 1 et 50, de préférence entre 2 et 30. De manière avantageuse, les tensioactifs non ioniques ne comprennent pas de motifs oxypropylénés.

Conformément à un mode de réalisation préféré de l'invention, les tensioactifs non ioniques oxyalkylénés sont choisis parmi les alcools en C₈-C₃₀, oxyéthylénés, de préférence en C₁₈-C₃₀, oxyéthylénés

A titre d'exemple de tensioactifs non ioniques mono- ou poly-glycérolés, on utilise de préférence les alcools en C₈-C₄₀, mono- ou poly-glycérolés.

En particulier, les alcools en C₈-C₄₀ mono- ou poly- glycérolés correspondent à la formule suivante :

RO-[CH₂-CH(CH₂OH)-O]ₘ-H

dans laquelle R représente un radical alkyle ou alcényle, linéaire ou ramifié, en C₈-C₄₀, de préférence en C₈-C₃₀, et m représente un nombre allant de 1 à 30 et de préférence de 1 à 10.

A titre d'exemple de composés convenables dans le cadre de l'invention, on peut citer, l'alcool laurique à 4 moles de glycérol (nom INCI : POLYGLYCERYL-4 LAURYL ETHER), l'alcool laurique à 1,5 moles de glycérol, l'alcool oléique à 4 moles de glycérol (nom INCI : POLYGLYCERYL-4 OLEYL ETHER), l'alcool oléique à 2 moles de glycérol (Nom INCI : POLYGLYCERYL-2 OLEYL ETHER), l'alcool cétéarylique à 2 moles de glycérol, l'alcool cétéarylique à 6 moles de glycérol, l'alcool oléocétylique à 6 moles de glycérol, et l'octadécanol à 6 moles de glycérol.

L'alcool peut représenter un mélange d'alcools au même titre que la valeur de m représente une valeur statistique, ce qui signifie que dans un produit commercial peuvent coexister plusieurs espèces d'alcools gras polyglycérolés sous forme d'un mélange.

Parmi les alcools mono- ou poly-glycérolés, on préfère plus particulièrement utiliser l'alcool en C₈/C₁₀ à une mole de glycérol, l'alcool en C₁₀/C₁₂ à 1 mole de glycérol et l'alcool en C₁₂ à 1,5 mole de glycérol.

De préférence, le tensioactif présent dans la composition de l'invention est un tensioactif non ionique.

La teneur en tensioactifs dans la composition de l'invention représente plus particulièrement de 0,1 à 50 % en poids, de préférence de 0,5 à 30 % en poids par rapport au poids de la composition.

Bien entendu, l'homme de l'art veillera à choisir le(s) éventuel(s) adjuvant(s) mentionné(s) ci-avant(s), de manière telle que les propriétés avantageuses attachées intrinsèquement aux compositions selon l'invention ne soient pas, ou substantiellement pas, altérées par l' adjonction (les adjonctions) envisagée(s).

Le pH de la composition conforme à l'invention est généralement compris entre 3 et 12 environ, de préférence entre 5 et 11 environ, préférentiellement 7 à 11. Il peut être ajusté à la valeur désirée au moyen d'agents acidifiants ou alcalinisants habituellement utilisés en teinture des fibres kératiniques ou bien encore à l'aide de systèmes tampons classiques.

Les agents alcalins sont par exemple ceux décrits précédemment.

Parmi les agents acidifiants, on peut citer, à titre d'exemple, des acides minéraux ou organiques comme l'acide chlorhydrique, l'acide ortho-phosphorique, des acides carboxyliques comme l'acide tartrique, l'acide citrique, l'acide lactique, ou des acides sulfoniques.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique sur les fibres la composition selon la présente invention telle que définie précédemment. La couleur peut être révélée à pH acide, neutre ou alcalin et l'agent oxydant peut être ajouté juste au moment de l'emploi ou il peut être mis en oeuvre simultanément ou séquentiellement aux autres composés de la composition de l'invention.

Après un temps de pose variant généralement de 1 à 60 minutes environ, de préférence 5 à 45 minutes environ, les fibres kératiniques sont rincées, éventuellement lavées au shampooing et rincées à nouveau, puis séchées.

La composition selon l'invention peut résulter du mélange d'au moins deux compositions et de préférence de 2 ou 3 compositions dont préférentiellement une composition oxydante comprenant au moins un agent oxydant tel que défini précédemment. Une des compositions peut être anhydre.

L'invention a aussi pour objet un dispositif à plusieurs compartiments ou "kit" de teinture dans lequel un premier compartiment contient une composition comprenant un ou plusieurs corps gras, un second compartiment comprenant un ou plusieurs précurseurs de colorant et éventuellement un ou plusieurs agents alcalins et un troisième compartiment comprenant un ou plusieurs agents oxydants, ce troisième compartiment pouvant contenir une partie des corps gras , un ou plusieurs polymères épaississants se trouvant dans au moins un des trois compartiments. Dans ce mode de réalisation, la composition comprenant le ou les corps gras peut être anhydre. On entend, par composition anhydre, au sens de l'invention, une composition cosmétique présentant une teneur en eau inférieure à 5 % en poids, de préférence inférieure à 2% en poids et de manière encore plus préférée inférieure à 1% en poids par rapport au poids de ladite composition. Il est à noter qu'il s'agit plus particulièrement d'eau liée, comme l'eau de cristallisation des sels ou des traces d'eau absorbée par les matières premières utilisées dans la réalisation des compositions selon l'invention. De préférence le ou les polymères épaississants se trouvent dans les second ou troisième compartiments et encore plus préférentiellement dans le second compartiment

Selon un second mode de réalisation, le dispositif de l'invention comprend un premier compartiment qui contient une composition comprenant un ou plusieurs corps gras et un ou plusieurs agents oxydants et un second compartiment comprenant un ou plusieurs précurseurs de colorant et éventuellement un ou plusieurs agents alcalins , un ou plusieurs polymères épaississants se trouvant dans au moins un des deux compartiments et de préférence dans le second compartiment .

Selon un troisième mode de réalisation, le dispositif de l'invention comprend un premier compartiment contenant une composition comprenant un ou plusieurs corps gras, un ou plusieurs précurseurs de colorant, éventuellement un ou plusieurs agents alcalins et un second compartiment contenant un ou plusieurs agents oxydants , un ou plusieurs polymères épaississants se trouvant dans au moins un des deux compartiments et de préférence dans le second compartiment.

Ces dispositifs peuvent être équipés d'un moyen permettant de délivrer sur les cheveux le mélange souhaité, tel que les dispositifs décrits dans le brevet FR-A-2 586 913 au nom de la Demanderesse.

La présente invention a également pour objet l'utilisation pour la teinture d'oxydation des fibres kératiniques, et en particulier des fibres kératiniques humaines telles que les cheveux, d'une composition tinctoriale telle que définie précédemment.

Les exemples qui suivent sont destinés à illustrer l'invention sans pour autant en limiter la portée.

### EXEMPLES

Les compositions suivantes ont été préparées (concentrations indiquées pour les produits en l'état ):

### Exemple 1

| **Composition 1** | **Concentration (g%)** |
|---|---|
| DISTEARDIMONIUM HECTORITE | 3 |
| OCTYLDODECANOL | 11,5 |
| GLYCOL DISTEARATE | 8 |
| HUILE DE VASELINE | 64,5 |
| PROPYLENE CARBONATE | 1 |
| LAURETH-2 | 1 |
| POLYSORBATE 21 | 11 |

| **Composition 2** | **concentration (g%)** |
|---|---|
| ACIDE DIETHYLENE TRIAMINE PENTACETIQUE, SEL PENTASODIQUE EN SOLUTION AQUEUSE A 40 % | 1 |
| METABISULFITE DE SODIUM | 0,7 |
| MONOETHANOLAMINE | 14,5 |
| 1-METHYL-2,5-DIAMINOBENZENE | 2,25 |
| 2,4-DIAMINOPHENOXYETHANOL HCl | 0,05 |
| 1,3-DIHYDROXYBENZENE (RESORCINOL) | 2 |
| m-AMINOPHENOL | 0,36 |
| NATROSOL 250 HHR (hydroxyethylcellulose) | 1,5 |
| HEXYLENE GLYCOL | 3 |
| DIPROPYLENE GLYCOL | 3 |
| ALCOOL ETHYLIQUE | 8,25 |
| PROPYLENEGLYCOL | 6,2 |
| ACIDE ASCORBIQUE | 0,25 |
| EAU | Qs 100 g |

| **Composition 3** | **Concentration (g%)** |
|---|---|
| ACIDE DIETHYLENE TRIAMINE PENTACETIQUE, SEL PENTASODIQUE EN SOLUTION AQUEUSE A 40 % | 0,15 |
| PEROXYDE D'HYDROGENE EN SOLUTION A 50 % (EAU OXYGENEE 200 VOL.) | 12 |
| STANNATE DE SODIUM | 0,04 |
| PYROPHOSPHATE DE SODIUM | 0,03 |
| HUILE DE VASELINE | 20 |
| HEXADIMETHRINE CHLORIDE (MA à 60% ans l'eau) | 0,25 |
| POLYQUATERNIUM-6 (MA à 40% dans l'eau) | 0,5 |
| GLYCERINE | 0,5 |
| ALCOOL CETYLSTEARYLIQUE (C16/C18 30/70) | 8 |
| ALCOOL CETYLSTEARYLIQUE OXYETHYLENE (33 OE) | 3 |
| AMIDE D'ACIDES DE COLZA OXYETHYLENE (4 OE) PROTEGE à 92.3 % dans l'eau | 1,3 |
| VITAMINE E | 0,1 |
| ACIDE PHOSPHORIQUE | Qs pH 2.2 |
| EAU | QS 100 g |

### Exemple 2

| **Composition 2'** | **concentration (g%)** |
|---|---|
| ACIDE DIETHYLENE TRIAMINE PENTACETIQUE, SEL PENTASODIQUE EN SOLUTION AQUEUSE A 40 % | 1 |
| METABISULFITE DE SODIUM | 0,7 |
| MONOETHANOLAMINE | 14,5 |
| 1-METHYL-2,5-DIAMINOBENZENE | 2,25 |
| 2,4-DIAMINOPHENOXYETHANOL HCI | 0,05 |
| 1,3-DIHYDROXYBENZENE (RESORCINOL) | 2 |
| m-AMINOPHENOL | 0,36 |
| NATROSOL PLUS 330 CS (cétylhydroxyethylcellulose) | 2 |
| DIPROPYLENE GLYCOL | 3 |
| HEXYLENE GLYCOL | 3 |
| PROPYLENEGLYCOL | 6,2 |
| | |
| ACIDE ASCORBIQUE | 0,25 |
| EAU | Qs 100 g |

Les compositions 1' et 3' mises en oeuvre dans cet exemple sont respectivement identiques aux compositions 1 et 3 décrites à l'exemple

### Exemple 3

| **Composition 1"** | **Concentration (g%)** |
|---|---|
| OCTYLDODECANOL | 14,5 |
| INTELIMER IPA 13-6 | 5 |
| HUILE DE VASELINE | 74 |
| OLETH-10 | 6,5 |

Les compositions 2" et 3" mises en oeuvre dans cet exemple sont respectivement identiques aux compositions 2 et 3 décrites à l'exemple 1.

Les compositions 1, 2, 3 ou 1',2' et 3' ou 1", 2", 3" de chacun des exemples sont mélangées au moment de l'emploi dans les proportions suivantes : 10 g de la composition 1( ou 1' ou 1") avec 4 g de la composition 2 (ou 2' ou 2") et 16 g de la composition 3(ou 3' ou 3"). Le mélange est appliqué sur des mèches de cheveux gris naturels à 90 % de cheveux blancs à raison de 10 g de mélange pour 1 g de cheveux. Après 30 minutes de pause, les cheveux sont rincés, lavés avec un shampooing standard et séchés.

La coloration capillaire est évaluée de manière visuelle.

| | |
|---|---|
| **Exemple 1** | Châtain clair |
| | Naturel |
| **Exemple 2** | Châtain clair |
| | Naturel |
| **Exemple 3** | Châtain clair |
| | Naturel |

## Revendications

1. Composition pour la teinture d'oxydation des fibres kératiniques, comprenant A) au moins 25 % d'un ou plusieurs corps gras différents des acides gras, B) un ou plusieurs polymères épaississants, C) un ou plusieurs précurseurs de colorant, D) un ou plusieurs agents oxydants et E) un ou plusieurs agents alcalins dont au moins un de ces agents alcalins est une amine organique.

2. Composition selon la revendication 1 **caractérisé en ce que** le ou les corps gras sont choisis parmi les alcanes inférieurs, les alcools gras, les esters d'acides gras, les esters d'alcool gras, les huiles non siliconées, les cires non siliconées et les silicones.

3. Composition selon l'une quelconque des revendications précédentes dans laquelle le corps gras est non siliconé.

4. Composition selon l'une quelconque des revendications précédentes dans laquelle le polymère épaississant est un polymère épaississant pour phase aqueuse.

5. Composition selon l'une quelconque des revendications précédentes dans laquelle le polymère épaississant est choisi parmi les polymères à motifs sucre associatifs ou non et les polymères anioniques acryliques ou méthacryliques associatifs ou non, les polyuréthanes associatifs ou non.

6. Composition selon l'une quelconque des revendications 1 à 3 dans laquelle le polymère épaississant est un polymère épaississant pour phase grasse.

7. Composition selon l'une quelconque des revendications précédentes dans laquelle le polymère épaississant est choisi parmi les polymères portant dans le squelette au moins une séquence cristallisable , les polyamides non siliconés, les polyamides siliconés Les mono- ou polyalkylesters de saccharide ou polysaccharide les dérivés amides d'aminoacides N-acylés et les copolymères comprenant une séquence alkylène ou styrène et de préférence parmi les polymères portant dans le squelette au moins une séquence cristallisable.

8. Composition selon l'une quelconque des revendications précédentes, **caractérisée en ce** le ou les polymères épaississants est ou sont présents dans la composition selon l'invention dans une teneur allant de 0,01 à 10 % en poids et de préférence de 0,1 à 5 % en poids par rapport au poids total de la composition.

9. Composition selon l'une quelconque des revendications précédentes, **caractérisée en ce que** le précurseur de colorant est choisi parmi les bases d'oxydation et les coupleurs.

10. Composition selon la revendication précédente, **caractérisée en ce que** le précurseur de colorant est choisi parmi les bases d'oxydation ortho- et para-phénylènediamines, les bases doubles, les ortho- et para-aminophénols, les bases hétérocycliques, ainsi que les sels d'addition de ces composés avec un acide et les coupleurs méta-aminophénols, les méta-phénylènediamines, les méta-diphénols, les naphtols, les coupleurs hétérocycliques et les sels d'addition de ces composés avec un acide, de préférence aminophénols et/ou méta-phénylènediamines.

11. Composition selon l'une quelconque des revendications précédentes, **caractérisée en ce que** l'agent oxydant est un peroxyde, de préférence le peroxyde d'hydrogène.

12. Composition selon l'une quelconque des revendications précédentes dans laquelle l'agent alcalin est l'ammoniac et /ou une alcanolamine.

13. Composition selon l'une quelconque des revendications précédentes dans laquelle l'agent alcalin est la monoéthanolamine.

14. Procédé de teinture des fibres kératiniques, **caractérisé en ce qu'**une composition telle que définie à l'une quelconque des revendications 1 à 13 est appliquée sur les fibres kératiniques pendant une durée suffisante pour développer la coloration désirée.

15. Dispositif à plusieurs compartiments comprenant un premier compartiment contient une composition comprenant un ou plusieurs corps gras, un second compartiment comprenant un ou plusieurs précurseurs de colorant et un ou plusieurs agents alcalins et un troisième compartiment comprenant un ou plusieurs agents oxydants, ce troisième compartiment pouvant contenir une partie des corps gras ; un ou plusieurs polymères épaississants se trouvant dans au moins un des trois compartiments ; le ou les corps gras, le ou les précurseurs de colorant, le ou les agents oxydants, le ou les agents alcalins, le ou les polymères épaississants étant définis selon l'une quelconque des revendications 1 à 13.

16. Dispositif à plusieurs compartiments comprenant un premier compartiment qui contient une composition comprenant un ou plusieurs corps gras et un ou plusieurs agents oxydants et un second compartiment comprenant un ou plusieurs précurseurs de colorant et un ou plusieurs agents alcalins un ou plusieurs polymères épaississants se trouvant dans au moins un des deux compartiments et de préférence dans le second compartiment ; le ou les corps gras, le ou les précurseurs de colorant, le ou les agents oxydants, le ou les polymères épaississants et le ou les agents alcalins étant définis selon l'une quelconque des revendications 1 à 13.

17. Dispositif à plusieurs compartiments comprenant un premier compartiment contenant une composition comprenant un ou plusieurs corps gras, un ou plusieurs précurseurs de colorant, un ou plusieurs agents alcalins et un second compartiment contenant un ou plusieurs agents oxydants ; un ou plusieurs polymères épaississants se trouvant dans au moins un des deux compartiments et de préférence dans le second compartiment, le ou les corps gras, le ou les précurseurs de colorant, le ou les agents oxydants, le ou les polymères épaississants et le ou les agents alcalins étant définis selon l'une quelconque des revendications précédentes 1 à 13.

18. Utilisation pour la teinture d'oxydation des fibres kératiniques d'une composition telle que définie à l'une quelconque des revendications 1 à 13.
